# EUROPEAN PATENT APPLICATION

(11) **EP 4 400 432 A2**
(43) Date of publication of application: **17.07.2024**
(21) Application number: 24178668.0
(22) Date of filing: 29.03.2022
(51) Int. Cl.: B65B 3/00

(54) **SYSTEM FOR LOW-TEMPERATURE STORAGE OF A PHARMACEUTICAL COMPOSI-TION, LIQUID COMPOSITION, METHOD AND USES**

(30) Priority: 30.03.2021 US 202163167869 P; 13.09.2021 US 202163243477 P; 13.09.2021 US 202163243485 P; 13.09.2021 US 202163243558 P; 13.01.2022 US 202263266734 P
(62) Divisional of application: 22718226.8
(71) Applicant: SCHOTT Pharma Schweiz AG, 9001 St. Gallen (CH); Schott North America, Inc., Rye Brook, NY 10573 (US)
(72) Inventor: BRECHLER, Sebastian, 9001 St. Gallen (CH); EGLI, Rahel, 9001 St. Gallen (CH); HEIZ, Benjamin, 9001 St.Gallen (CH); MOSER, Raymond, 9001 St. Gallen (CH); VAN GINNEKEN, Tom, 9404 Rorschacherberg (CH); GALLAGHER, Patrick, Lebanon, PA, 17042 (US); BAUERT, Dominique, St. Gallen, 9001 (CH); STERN, Matthias, 9001 St. Gallen (CH)
(74) Representative: Schott Corporate IP

(57) **Abstract**

The present invention relates to a system for low-temperature storage of a pharmaceutical composition. The invention also relates to a liquid composition, a method and uses.

## Description

This application claims priority to U.S. Provisional Patent Application No. 63/167,869 filed on March 30, 2021, U.S. Provisional Patent Application No. 63/243,477 filed on September 13, 2021, U.S. Provisional Patent Application No. 63/243,485 filed on September 13, 2021, U.S. Provisional Patent Application No. 63/243,558 filed on September 13, 2021, and U.S. Provisional Patent Application No. 63/266,734 filed on January 13, 2022, which all are incorporated herein by reference in their entirety.

The present invention relates to a system for low-temperature storage of a pharmaceutical composition. The invention also relates to a liquid composition, a method and uses.

Pharmaceutical containers for drug delivery are as such known in the prior art. These containers usually feature a stopper useful for eluting the contents of the container through an outlet and a closure. The stopper must slide within the container and yet provide for a tight seal so that the composition remains safely stored even for extended time periods.

However, if such a pharmaceutical container prefilled with a pharmaceutical composition is stored at low temperatures, there is a risk that the tight seal is compromised during cooling or thawing.

It is, therefore, an object of the present invention to provide means that overcome the problems of the prior art and which can be used also for storing compositions at low temperatures. A further object of the invention is to provide a composition, a method and uses.

### Description of the invention

The object is solved by the invention according to a first aspect in that a system for storing a pharmaceutical composition at low temperatures, the system comprising
- a pharmaceutical container for storing a pharmaceutical composition at low temperatures, the container having a barrel, a stopper, which fluidally closes a first end of the barrel, and a closure, which fluidally closes a second end of the barrel;
- a liquid pharmaceutical composition and a gas, both, arranged within the barrel of the container between the stopper and the closure,
is proposed.

In one embodiment the pharmaceutical composition comprises or is a liquid.

For example, the material of the barrel can be or can comprise Cycloolefin Copolymer (COC). Alternatively or in addition, the coefficient of linear thermal expansion of the material of the barrel may be between 10⁻³ K⁻¹ and 10⁻⁴ K⁻¹, especially 0.6 10⁻⁴ K⁻¹. The coefficient of linear thermal expansion may be measured according to ISO 11359, part 1/2.

In one embodiment, the system is held at a storage temperature which is equal to or below zero degrees C, especially equal to or below the freezing point of the pharmaceutical composition.

In one embodiment the pharmaceutical container is a syringe. This is particular preferred because this way the pharmaceutical composition can be stored directly in the syringe at low temperatures in form of a pre-filled syringe. This makes it very easy to handle the composition because the syringe is prefilled and can be used for administration without transferring the composition to a different container. All the more, this is even possible if the storage temperature of the composition is below a temperature where the expansion of the composition takes place.

Throughout this application, room temperature is preferably a temperature of 293.15 K, especially at 101.325 kPa.

For example, at least part of the second end may be in form of or comprise a threaded portion or a Luer Lock.

For example, the closure may be in form of or comprise a threaded portion, a Luer Lock, a Tip Cap and/or a Rigid Cap. In one embodiment the closure is in addition or as an alternative connected to the barrel in a reclosable manner. In one embodiment the closure is in addition or as an alternative removable from the barrel in a non-destructive manner.

In one embodiment it might be preferred that, especially for a particular orientation of the container, a volume enclosed by the barrel which is occupied by the gas is defined as headspace of the system.

In one embodiment it might be preferred that, for a vertical orientation of the container with the first end being at the bottom and the second end being at the top, the volume enclosed by the barrel between the stopper and the closure which is occupied by the gas is defined as headspace A of the system

In one embodiment it might be preferred that, for a vertical orientation of the container with the second end being at the bottom and the first end being at the top, the volume enclosed by the barrel between the stopper and the closure which is occupied by the gas is defined as headspace B of the system.

The headspace, such as the headspace A and/or the headspace B, may be variable depending on the ambient temperature, the ambient pressure or the like. This is because the headspace, such as the headspace A and/or the headspace B, corresponds to the volume of the gas within the barrel which volume may be subject to changes. For example, the volume of the gas depends on the environmental conditions and/or on the volume of the composition within the barrel.

In one embodiment it might be preferred that, at room temperature, the volume occupied by the headspace, such as the headspace A and/or the headspace B, is 1 % or more of the volume occupied by the composition.

This is, thus, based on the surprising finding that the storage of a composition even at low temperatures is possible if a gas volume of appropriate size is provided within the container. It turned out that this allows a change of the volume of the composition without affecting the integrity of the container during cooling or thawing. This is because the proposed headspace, such as the headspace A and/or the headspace B, makes the system resistant to the expansion or retraction of the pharmaceutical composition when the composition is cooled, especially to or below zero degrees C or to or below the freezing point of the composition. The same applies when the system is thawed and the pharmaceutical composition expands or retracts as well. Furthermore, a deterioration of material characteristics is prevented by the proposed system due to reduced impacts during the cooling or thawing process.

In other words, the system allows that a prefilled container can be used and be frozen along with the composition. At the same time the risk is significant reduced that due to a change of volume of the composition during cooling or thawing, the stopper moves or the container leaks. This is very useful for pharmaceutical compositions which need to be stored at low temperatures. Especially if the storage temperature is below the freezing point of the composition.

In addition, during cooling below the freezing point, the space the container requires within an additional packaging is compact and may not change. In addition, also safety is maintained because even at low temperatures the container remains closed.

The advantages are achieved in that the force applied to the stopper is reduced in that the headspace, such as the headspace A and/or the headspace B, is variable. Or in other words, in that the volume of the gas can be changed, e.g. compressed or expanded, by the space the composition occupies so that the force applied to the stopper or the barrel is reduced. Hence, an uncontrolled movement of the stopper or damaging of the barrel can be prevented. This prevents a leakage of the pharmaceutical composition caused by the release of the stopper from the barrel. Hence, the expansion of the composition can take place within the volume occupied by the gas rather than by moving the stopper. The expansion can, thus, be controlled.

This is an important finding because at lower temperatures two aspects have to be considered. First, the break loose force of the stopper within the barrel may change due to a change of properties of the material of the stopper and the container. Second, the force acting on the stopper may change, especially increase at least for some temperature ranges, due to different volumes occupied by the composition at different temperatures.

For the system having a proposed headspace, such as the headspace A and/or the headspace B, at room temperature, the stopper may provide a sufficient resistance to expansion of the pharmaceutical composition and still functions even at low temperatures, e.g at temperatures below 4 degrees C or even below zero degrees C, below -20°C, below -40 °C or below -60°C. Hence, a pharmaceutical composition held by the barrel can be forced to expand within another available space rather than moving the stopper.

The proposed system is, thus, well-suited for low-temperature storage of pharmaceutical compositions.

The break loose force can be regarded as the force acting, especially axial, on the stopper in a direction away from the pharmaceutical composition, which is required to initiate a movement of the stopper within the barrel. Once such a movement is initiated and to keep the movement going, only a gliding force acts on the stopper, which gliding force is smaller than the break loose force. The break loose force may insofar be regarded as a measure for "how firmly the stopper rests within the barrel".

In one embodiment it might be preferred that the headspace B has a cylindrical volume domain portion which volume domain portion has a specific diameter equal to the inside diameter of the barrel and a specific height, which specific height preferably is measured from the central point of the stopper to the surface of the liquid pharmaceutical composition facing the stopper, wherein the specific height has a value of 0.1 mm or more.

It is the surprising finding that a specific height chosen accordingly leads to the beneficial situation that the interaction between an expansion of the composition and a compression of the gas is such that only reduced or even no movement of the stopper is observed during the freezing and thawing process. The inventors have found that the specific height of at least 0.1 mm constitutes an optimal basis for said interaction.

It is noted that for a stopper with the surface facing the liquid composition being a flat surface, the volume of the cylindrical volume domain portion might be equal to the volume of the headspace B.

Preferably, when measuring the specific height, the container has a vertical orientation with the second end being at the bottom and the first end being at the top..

In one embodiment it might be preferred that the specific height is
(i) 0.2 mm or more, preferably 0.5 mm or more, preferably 0.7 mm or more, preferably 1 mm or more, preferably between 2 mm or more, preferably 3 mm or more, preferably 4 mm or more, preferably 5 mm or more, preferably 6 mm or more, preferably 7 mm or more, preferably 8 mm or more, preferably 9 mm or more, preferably 10 mm or more, preferably 11 mm or more, preferably 12 mm or more, preferably 13 mm or more, preferably 14 mm or more, preferably 15 mm or more,
(ii) 15 mm or less, preferably 14 mm or less, preferably 13 mm or less, preferably 12 mm or less, preferably 11 mm or less, 10 mm or less, preferably 9 mm or less, preferably 8 mm or less, preferably 7 mm or less, preferably 6 mm or less, preferably 5 mm or less, preferably 4 mm or less, preferably 3 mm or less, preferably 2 mm or less,
   and/or
(iii) between 1 mm and 15 mm, preferably between 1 mm and 10 mm, preferably between 1 mm and 8 mm, preferably between 1 mm and 3 mm, such as 2 mm, or between 3 mm and 5 mm, such as 4 mm, or between 5 mm and 7 mm, such as 6 mm, or between 7 mm and 9 mm, such as 8 mm.

The specific height can further be specified dependent on the situation the system is used for. The proposed specific height provided preferred results concerning a reduced or even eliminated stopper movement during the freezing and thawing process.

In one embodiment it might be preferred that the ratio [mm/mm] of the inside diameter of the barrel and the specific height is
(i) 0.3 or more, preferably 0.7 or more, preferably 1 or more, preferably 1.05 or more, preferably 1.1 or more, preferably 1.3 or more, preferably 1.5 or more, preferably 2 or more, preferably 2.5 or more, preferably 3 or more, preferably 3.5 or more, preferably 4 or more, preferably 4.5 or more, preferably 5 or more, preferably 7 or more, preferably 10 or more,
(ii) 10 or less, preferably 6 or less, preferably 5 or less, preferably 4 or less, preferably 3 or less, preferably 2 or less, preferably 1.5 or less, preferably 1.3 or less, preferably 1.0 or less, preferably 0.7 or less, preferably 0.5 or less,
   and/or
(iii) between 0.3 and 10, preferably between 1 and 10, preferably between 1 and 5, preferably between 1 and 2 or between 2 and 4 or between 3 and 5..

The ratio links geometric aspects of the system, i.e. the inside diameter of the barrel, with aspects originally not relating to geometric shapes of the system. However, a ratio chosen in line with the proposed values turned out to provide a reliable basis for providing a system having no or only little stopper movement during freezing / thawing.

In one embodiment it might be preferred that the total length of the barrel, especially measured along the axial extension of the barrel, is
(i) 40 mm or more, preferably 45 mm or more, preferably 50 mm or more, preferably 55 mm or more, preferably 60 mm or more, preferably 65 mm or more, preferably 70 mm or more, preferably 75 mm or more, preferably 80 mm or more,
(ii) 80 mm or less, preferably 75 mm or less, preferably 70 mm or less, preferably 65 mm or less, preferably 60 mm or less, preferably 55 mm or less, preferably 50 mm or less, preferably 45 mm or less, preferably 40 mm or less,
   and/or
(iii) between 40 mm and 80 mm, preferably between 40 mm and 60 mm, especially between 45 mm and 55 mm, particularly between 45 mm and 50 mm, or between 60 mm and 70 mm, particularly between 63 mm and 67 mm..

For example, the total length of the barrel can be 65.7 mm or 48.4 mm.

In one embodiment it might be preferred that the inside diameter of the barrel is
(i) 5 mm or more, preferably 6 mm or more, preferably 7 mm or more, preferably 8 mm or more, preferably 9 mm or more,
(ii) 10 mm or less, preferably 9 mm or less, preferably 8 mm or less, preferably 7 mm or less, preferably 6 mm or less, preferably 5 mm or less,
   and/or
(iii) between 5 mm and 10 mm, preferably between 5 mm and 7 mm, especially between 6 mm and 7 mm, particularly between 6 mm and 6.5 mm such as 6.35 mm, or between 7 mm and 9 mm, especially between 8 mm and 9 mm, particularly between 8.5 mm and 9 mm such as 8.65 mm..

For example, the inside diameter of the barrel can be 6.35 mm or 8.65 mm.

In one embodiment it might be preferred that
(i) the total length of the barrel, especially measured along the axial extension of the barrel, is between 45 mm and 50 mm and the inside diameter of the barrel is between 8 mm and 9 mm,
   or
(ii) the total length of the barrel, especially measured along the axial extension of the barrel, is between 60 mm and 70 mm and the inside diameter of the barrel is between 5 mm and 7 mm..

A respective chosen combination of the total length of the barrel and the inside diameter of the barrel lead to particular preferred systems.

In one embodiment it might be preferred that the following equation(s) is/are fulfilled:
(X/Y)/Z≤V; and/or
W≤(X/Y)/Z;
wherein X is the volume (ml) of the pharmaceutical composition at room temperature;
wherein Y is the volume (ml) of the headspace at room temperature;
wherein Z is the inner diameter (mm) of the barrel;
wherein V (1/mm) is 1.5, preferably 1.4, preferably 1.3, preferably 1.2, preferably 1.1, preferably 1.0, preferably 0.9, preferably 0.8, preferably 0.7, preferably 0.6, preferably 0.5, preferably 0.4, preferably 0.3, preferably 0.2, preferably 0.1;
and/or
wherein W (1/mm) is 0.01, preferably 0.05, preferably 0.1, preferably 0.2, preferably 0.3, preferably 0.4, preferably 0.5, preferably 0.6, preferably 0.7.

In one embodiment it might be preferred that the liquid pharmaceutical composition comprises an additive.

It is the surprising finding that adding an additive to the composition improves the stopper movement during freezing and/or thawing the composition.

In one embodiment it might be preferred that the additive is selected from a group consisting of salt, sugar, lipid and acid.

In one embodiment it might be preferred that the additive is selected from a group consisting of NaCl, KCI, sucrose, sodium acetate, acetic acid, ((4-hydroxybutyl)azanediyl)bis(hexan-6,1-diyl)bis(2-hexyldecanoat), 2[(polyethylene glycol)-2000]-N,N-ditetradecylacetamide, 1,2-Distearoyl-sn-glycero-3-phosphocholine, SM(sphyngomyelin)-102, polyethylene glycol [PEG] 2000 dimyristoyl glycerol [DMG], 1,2-distearoyl-sn-glycero-3-phosphocholine [DSPC], cholesterol, polyethylene glycol (PEG), H₃PO₄, XH₂PO₄, X₂HPO₄ and X₃PO₄, wherein X is Na and/or K.

In one embodiment it might be preferred that the additive is NaCl.

In one embodiment it might be preferred that the additive is KCI

In one embodiment it might be preferred that the additive is sucrose.

In one embodiment it might be preferred that the additive is sodium acetate.

In one embodiment it might be preferred that the additive is acetic acid.

In one embodiment it might be preferred that the additive is a lipid, preferably ((4-hydroxybutyl)azanediyl)bis(hexan-6,1-diyl)bis(2-hexyldecanoat), 2[(polyethylene glycol)-2000]-N,N-ditetradecylacetamide, 1,2-Distearoyl-sn-glycero-3-phosphocholine, SM(sphyngomyelin)-102, polyethylene glycol [PEG] 2000 dimyristoyl glycerol [DMG], 1,2-distearoyl-sn-glycero-3-phosphocholine [DSPC] and/or cholesterol.

In one embodiment it might be preferred that the additive is polyethylene glycol (PEG).

In one embodiment it might be preferred that the additive is H₃PO₄.

In one embodiment it might be preferred that the additive is XH₂PO₄, wherein X is Na and/or K.

In one embodiment it might be preferred that the additive is X₂HPO₄, wherein X is Na and/or K.

In one embodiment it might be preferred that the additive is X₃PO₄, wherein X is Na and/or K.

In one embodiment it might be preferred that the additive is cholesterol.

In one embodiment it might be preferred that the concentration of the additive is:
(i) 0.01 mol/l or more, preferably 0.05 mol/l or more, preferably 0.1 mol/l or more, preferably 0.2 mol/l or more, preferably 0.3 mol/l or more, preferably 0.4 mol/l or more, preferably 0.5 mol/l or more, preferably 0.7 mol/l or more, preferably 1 mol/l or more,
(ii) 1 mol/l or less, preferably 0.9 mol/l or less, preferably 0.8 mol/l or less, preferably 0.7 mol/l or less, preferably 0.6 mol/l or less, preferably 0.5 mol/l or less, preferably 0.4 mol/l or less, preferably 0.3 mol/l or less, preferably 0.2 mol/l or less,
   and/or
(iii) between 0.1 mol/l and 1 mol/l, preferably between 0.1 mol/l and 0.8 mol/l, preferably between 0.1 mol/l and 0.5 mol/l, preferably between 0.2 mol/l and 0.4 mol/l, preferably 0.25 mol/l.

Such concentrations turned out to lead to particularly preferred systems.

In one embodiment it might be preferred that the concentration of the additive is:
(i) 1 g/l or more, preferably 2g/l or more, preferably 5 g/l or more, preferably 10 g/l or more, preferably 20 g/l or more, preferably 30 g/l or more, preferably 40 g/l or more, preferably 50 g/l or more, preferably 75 g/l or more, preferably 100 g/l or more, preferably 150 g/l or more, preferably 200 g/l or more,
(ii) 300 g/l or less, preferably 200 g/l or less, preferably 150 g/l or less, preferably 120 g/l or less, preferably 100 g/l or less, preferably 90 g/l or less, preferably 80 g/l or less, preferably 70 g/l or less, preferably 60 g/l or less, preferably 50 g/l or less, preferably 40 g/l or less, preferably 30 g/l or less, preferably 20 g/l or less, preferably 10 g/l or less, preferably 5 g/l or less,
   and/or
(iii) between 1 g/l and 300 g/l, preferably between 5 g/l and 200 g/l, preferably between 20 g/l and 150 g/l, preferably between 50 g/l and 120 g/l, preferably between 60 g/l and 100 g/l, preferably between 70 g/l and 90 g/l.

Such concentrations turned out to lead to particularly preferred systems.

In one embodiment it might be preferred that the concentration of all compounds in the pharmaceutical composition is
(i) 0.01 mol/l or more, preferably 0.05 mol/l or more, preferably 0.1 mol/l or more, preferably 0.2 mol/l or more, preferably 0.3 mol/l or more, preferably 0.4 mol/l or more, preferably 0.5 mol/l or more, preferably 0.7 mol/l or more, preferably 1 mol/l or more,
(ii) 1 mol/l or less, preferably 0.9 mol/l or less, preferably 0.8 mol/l or less, preferably 0.7 mol/l or less, preferably 0.6 mol/l or less, preferably 0.5 mol/l or less, preferably 0.4 mol/l or less, preferably 0.3 mol/l or less, preferably 0.2 mol/l or less,
   and/or
(iii) between 0.1 mol/l and 1 mol/l, preferably between 0.1 mol/l and 0.8 mol/l, preferably between 0.1 mol/l and 0.5 mol/l, preferably between 0.2 mol/l and 0.4 mol/l, preferably 0.25 mol/l.

In one embodiment it might be preferred that, at room temperature, the headspace, such as the headspace A and/or the headspace B, is a volume which is
(i) 1.3 % or more, preferably 1.5 % or more, preferably 1.7 % or more, preferably 2 % or more, preferably 2.5 % or more, preferably 3 % or more, preferably 3.5 % or more, preferably 4 % or more, preferably 4.5 % or more, preferably 5 % or more, preferably 6 % or more, preferably 7 % or more, preferably 8 % or more, preferably 9% or more, preferably 10 % or more, preferably 11 % or more, preferably 12 % or more, preferably 13 % or more, preferably 14 % or more, preferably 15 % or more, preferably 17 % or more, preferably 20 % or more,
(ii) 20 % or less, preferably 17 % or less, preferably 15 % or less, preferably 14 % or less, preferably 13 % or less, preferably 12 % or less, preferably 11 % or less, preferably 10 % or less, preferably 9 % or less, preferably 8 % or less, preferably 7 % or less, preferably 6 % or less, preferably 5 % or less, preferably 4.5 % or less, preferably 4 % or less, preferably 3.5 % or less, preferably 3 % or less, preferably 2.5 % or less, preferably 2 % or less, preferably 1.7% or less, preferably 1.5 % or less, preferably 1.3 % or less,
   and/or
(iii) between 1 % and 20 %, especially between 1.5 % and 3 %, between 2 % and 5 %, between 4 % and 8 %, between 7 % and 12 %, between 10 % and 15 %, between 12 % and 17 % and/or between 15 % and 20 %,
respectively, of the volume occupied by the composition.

A larger headspace is preferred if the composition needs to be shaken prior to use. This is easier if the headspace is larger.

For example, for a barrel of nominal volume of 1 ml, the headspace, such as the headspace A and/or the headspace B, might be 2 mm along the axial direction of the container.

In one embodiment it might be preferred that
(i) the stopper being slidably arranged within the barrel,
(ii) the stopper having a circumferential surface at least partially contacting an inner surface of the barrel,
   and/or
(iii) the first end is a proximal end of the container, especially of the barrel, and/or the second end is a distal end of the container, especially of the barrel.

In one embodiment, for a vertical orientation, the stopper, the pharmaceutical composition and the gas may be arranged within the barrel from bottom to top.

In one embodiment it might be preferred that the system or parts thereof is/are designed in such a way, especially the headspace, such as the headspace A and/or the headspace B, at room temperature, the barrel, the stopper and/or the composition are adapted to each other in such a way, that in case of an expansion of the pharmaceutical composition, especially an expansion of the composition during cooling the system,
preferably (a) from a temperature above 4 degrees C, especially from room temperature, and/or (b) to or below zero degrees C, especially to or below the freezing point of the composition and/or to or below a storage temperature of the system,
(i) the pharmaceutical composition can expand or expands by displacing and/or compressing the gas, hence, reducing the volume of the headspace, such as the headspace A and/or the headspace B,,
(ii) the maximal force applied to the stopper by the pharmaceutical composition and/or the gas, especially in an axial direction, is lower than the break loose force of the stopper,
(iii) the stopper moves 5 mm or less, preferably 4 mm or less, preferably 3.5 mm or less, preferably 3 mm or less, preferably 2.7 mm or less, preferably 2.5 mm or less, preferably 2.3 mm or less, preferably 2.0 mm or less, preferably 1.7 mm or less, preferably 1.5 mm or less, preferably 1.3 mm or less, preferably 1.0 mm or less, preferably 0.8 mm or less, preferably 0.6 mm or less, preferably 0.5 mm or less, preferably 0.4 mm or less, preferably 0.3 mm or less, preferably 0.2 mm or less, preferably 0.1 mm or less,
   and/or
(iv) the stopper moves 0.01 mm or more, preferably 0.05 mm or more, preferably 0.1 mm or more, preferably 0.3 mm or more, preferably 0.5 mm or more, preferably 0.7 mm or more, preferably 1.0 mm or more,
wherein preferably the barrel is oriented in such a vertical orientation during cooling that (a) the first end is at the bottom and/or the second end is at the top and/or (b) the pharmaceutical composition and the gas are arranged along the axial direction of the container, especially of the barrel.

The headspace, such as the headspace A and/or the headspace B, can, thus, be reduced so that additional space can be accommodated by the expanded material of the composition. This reduces the risk that the container is damaged and/or the stopper moves.

The headspace, such as the headspace A and/or the headspace B, at a specific temperature, say room temperature, can easily be adapted, e.g. by changing the default position of the stopper within the barrel or by the size of the barrel. Hence, the system can be adapted in a convenient and efficient manner to withstand nearly any expected maximal force which might act on the stopper during cooling, thawing and storage of the system for a particular composition.

It might also be allowed that the stopper moves during expansion of the material of the composition. For example, this way the headspace, such as the headspace A and/or the headspace B, can be reduced.

If a movement of the stopper is allowed, it is preferred that the movement of the stopper is limited within certain boundaries. The proposed values are a preferred acceptable distance of an allowed movement. This is because the proposed distances correspond to typical distances between the two sealing lips of the stopper which have the greatest distance to each other, especially when the stopper is inserted inside the barrel and/or measured along the axial direction of the barrel.

At least one sealing lip typically serves as a sterile barrier between a sterile part of the inner surface of the barrel and a non-sterile part of the inner surface of the barrel. It is required that the pharmaceutical composition is only in contact with the sterile part of the inner surface of the barrel. Using the distance of such two lips as a reference is beneficial because if the movement of the stopper does not exceed the respective distance, the composition enclosed in the barrel has always only contact with the sterile part of the inner surface of the barrel.

Therefore, an adapted CCI test is defined elsewhere in the description which is passed by the system if the movement of the stopper is limited for a cooling-thawing cycle of the system. In one embodiment, the system passes the adapted CCI test as defined elsewhere in the description.

In one embodiment, the maximal movement may correspond to the distance between the two sealing lips of the stopper which have the greatest distance to each other, especially when the stopper is inserted inside the barrel and/or measured along the axial direction of the barrel. Alternatively or in addition, the maximal movement may correspond to the distance between two directly adjacent sealing lips of the stopper. This may further improve safety.

In one embodiment it might be preferred that
(i) the storage temperature is
   (a) -100 degrees C or above, preferably -80 degrees C or above, preferably -60 degrees C or above, preferably -40 degrees C or above, preferably -20 degrees C or above, preferably -10 degrees C or above,
   (b) 0 degrees C or below, -1 degrees C or below, preferably -5 degrees C or below, preferably -15 degrees C or below, preferably -25 degrees C or below, preferably -35 degrees C or below, preferably -55 degrees C or below, preferably -75 degrees C or below, preferably -95 degrees C or below,
      and/or
   (c) between -100 and 0 degrees C, preferably between -80 degrees C and -5 degrees C, preferably between -60 degrees C and -20 degrees C, preferably between -50 and -40 degrees C, and/or (d) 0 degrees C, -10 degrees C, -20 degrees C, -30 degrees C, -40 degrees C or -50 degrees C;
   and/or
(ii) the storage temperature is below the freezing temperature of the composition.

The storage temperature can be chosen in line with the requirements set forth by the pharmaceutical composition.

In one embodiment it might be preferred that
(i) the pharmaceutical composition comprises H₂O, especially more than 80 % (w/w), preferably more than 90 % (w/w), preferably more than 95 % (w/w), preferably more than 97 % (w/w), preferably more than 98 % (w/w), of the composition is H₂O;
   and/or
(ii) the gas comprises or is air, CO₂, N₂, Ar and/or O₂.

The pharmaceutical composition may comprise H₂O which is subject to a high expansion during the freezing process.

If the gas is air, CO₂, N₂, Ar and/or O₂, a particular cheap provision of the system is possible.

In one embodiment it might be preferred that the total length of the container, especially measured along the axial extension of the container, is
(i) 30 mm or more, preferably 40 mm or more, preferably 50 mm or more, preferably 60 mm or more, preferably 70 mm or more, preferably 80 mm or more, preferably 90 mm or more, preferably 100 mm or more, preferably 110 mm or more, preferably 120 mm or more, preferably 130 mm or more, preferably 150 mm or more, preferably 170 mm or more, preferably 190 mm or more,
(ii) 200 mm or less, preferably 150 mm or less, preferably 140 mm or less, preferably 130 mm or less, preferably 110 mm or less, preferably 100 mm or less, preferably 90 mm or less, preferably 80 mm or less, preferably 70 mm or less, preferably 60 mm or less, preferably 50 mm or less, preferably 40 mm or less,
   and/or
(iii) between 30 mm and 200 mm, preferably between 50 mm and 150 mm, especially between 50 mm and 100 mm, such as between 60 mm and 90 mm and/or between 90 mm and 150 mm.

For example, the total length of the container can be 73.9 mm, 74.6 mm, 92.4 mm, 72.7 mm, 55.4 mm, 59.9 mm, 62.5 mm, 74 mm, 91.7 mm, 72.6 mm, 54 mm, 72.5 mm, 90.5 mm, 85 mm, 105.8 mm, 128.3 mm or 136.9 mm.

For measuring the total length of the container, the closure may be in a state in which it closes the respective end of the barrel.

Preferably, for the present application, the term "between X and Y", with X and Y being certain values, is to be understood as to include the values X and Y. For example, a parameter having a value of between 1 and 2 means that the parameter might have a value which is between 1 and 2 inclusive of 1 and 2.

In one embodiment it might be preferred that the total length of the barrel, especially measured along the axial extension of the barrel, is
(i) 30 mm or more, preferably 40 mm or more, preferably 50 mm or more, preferably 60 mm or more, preferably 70 mm or more, preferably 80 mm or more, preferably 90 mm or more, preferably 100 mm or more, preferably 110 mm or more, preferably 120 mm or more, preferably 130 mm or more, preferably 150 mm or more, preferably 170 mm or more, preferably 190 mm or more,
(ii) 200 mm or less, preferably 150 mm or less, preferably 140 mm or less, preferably 130 mm or less, preferably 110 mm or less, preferably 100 mm or less, preferably 90 mm or less, preferably 80 mm or less, preferably 70 mm or less, preferably 60 mm or less, preferably 50 mm or less, preferably 40 mm or less,
   and/or
(iii) between 30 mm and 200 mm, preferably between 40 mm and 130 mm, especially between 40 mm and 80 mm, between 50 mm and 80 mm and/or between 70 mm and 130 mm.

For example, the total length of the barrel can be 65.7 mm, 67 mm, 84.7 mm, 65.7 mm, 48.4 mm, 52.9 mm, 55.5 mm, 67 mm, 84.7 mm, 64.5 mm, 45.9 mm, 64.4 mm, 82.4 mm, 76.9 mm, 97.7 mm, 120.2 mm or 128.8 mm.

In one embodiment it might be preferred that the inside diameter of the barrel is
(i) 5 mm or more, preferably 8 mm or more, preferably 10 mm or more, preferably 13 mm or more, preferably 15 mm or more, preferably 18 mm or more, preferably 20 mm or more, preferably 25 mm or more, preferably 30 mm or more,
(ii) 30 mm or less, preferably 28 mm or less, preferably 25 mm or less, preferably 20 mm or less, preferably 15 mm or less, preferably 13 mm or less, preferably 10 mm or less, preferably 8 mm or less, preferably 7 mm or less, preferably 6 mm or less,
   and/or
(iii) between 5 mm and 30 mm, preferably between 5 mm and 10 mm, between 10 mm and 15 mm, between 14 mm and 18 mm, between 17 mm and 20 mm and/or between 20 mm and 30 mm.

For example, the inside diameter of the barrel can be 6.35 mm, 8.65 mm, 6.5 mm, 8.75 mm, 12.2 mm, 14.7 mm, 18.2 mm or 27.9 mm.

In one embodiment it might be preferred that the outside diameter of the barrel is
(i) 5 mm or more, preferably 8 mm or more, preferably 10 mm or more, preferably 13 mm or more, preferably 15 mm or more, preferably 18 mm or more, preferably 20 mm or more, preferably 25 mm or more, preferably 30 mm or more, preferably 35 mm or more, preferably 40 mm or more,
(ii) 40 mm or less, preferably 35 mm or less, 30 mm or less, preferably 28 mm or less, preferably 25 mm or less, preferably 20 mm or less, preferably 15 mm or less, preferably 13 mm or less, preferably 10 mm or less, preferably 7 mm or less, preferably 5 mm or less,
   and/or
(iii) between 5 mm and 40 mm, preferably between 5 mm and 9 mm, between 8 mm and 13 mm, between 12 mm and 16 mm, between 15 mm and 20 mm, between 19 mm and 25 mm and/or between 25 mm and 35 mm.

For example, the outside diameter of the barrel can be 8.15 mm, 10.85 mm, 9.4 mm, 11.4 mm, 11.6 mm, 15 mm, 18 mm, 21.6 mm or 31.5 mm.

In one embodiment it might be preferred that at room temperature the composition has a volume of
(i) 0.1 ml or more, preferably 0.2 ml or more, preferably 0.3 ml or more, preferably 0.5 ml or more, preferably 0.7 ml or more, preferably 1 ml or more, preferably 1.5 ml or more, preferably 2 ml or more, preferably 3 ml or more, preferably 5 ml or more, preferably 10 ml or more, preferably 15 ml or more, preferably 20 ml or more, preferably 30 ml or more, preferably 50 ml or more, preferably 70 ml or more,
(ii) 100 ml or less, preferably 70 ml or less, preferably 55 ml or less, preferably 25 ml or less, preferably 15 ml or less, preferably 10 ml or less, preferably 7 ml or less, preferably 6 ml or less, preferably 5 ml or less, preferably 4 ml or less, preferably 3 ml or less, preferably 1 ml or less, preferably 0.7 ml or less, preferably 0.5 ml or less, preferably 0.3 ml or less, preferably 0.2 ml or less,
   and/or
(iii) between 0.1 ml and 100 ml, preferably between 0.1 ml and 1 ml or between 1 ml and 100 ml, such as between 1 ml and 50 ml, especially between 1 ml and 3 ml, between 1 ml and 10 ml, between 10 ml and 20 ml, between 20 ml and 30 ml, between 30 ml and 40 ml and/or between 40 ml and 50 ml.

For example, at room temperature the composition can have a volume of 1 ml, 1.25 ml, 2.25 ml, 3 ml, 5 ml, 10 ml, 20 ml or 50 ml.

In one embodiment it might be preferred that the position of the stopper moves 4.0 mm or less, preferably 3.0 mm or less, preferably 2.5 mm or less, preferably 2.0 mm or less, preferably 1.5 mm or less, preferably 1.0 mm or less, preferably 0.8 mm or less, preferably 0.5 mm or less, preferably 0.3 mm or less, preferably 0.1 mm or less, along the rotation axis of the barrel, when the system is cooled from room temperature (20°C) to -20°C, preferably -50°C, preferably - 80°C.

In one embodiment it might be preferred that the system has a temperature of -20°C or less and -200°C or more, preferably -40°C or less and -120°C or more, preferably -50°C or less and - 90°C or more.

In one embodiment it might be preferred that the system is stored for 5 days or more, preferably 1 month or more, preferably 3 month or more, preferably 6 month or more, preferably, 1 year or more.

In one embodiment it might be preferred that the stopper comprises at least one inner recess.

More precisely, the recess can be provided inside the stopper, preferably extending from one end of the stopper along a longitudinal axis of the stopper. In particular, the stopper can be provided with such inner recess both if used with a syringe barrel or if used with a cartridge barrel. Such a recess allows positioning of the stopper within the barrel in an easy and safe manner. When used with a syringe barrel, the inner recess can serve to receive a plunger rod.

In one embodiment it might be preferred that the barrel comprises a polymer, preferably cyclic olefin copolymers (COC) and/or cyclic olefin polymers (COP).

In one embodiment it might be preferred that the closure comprises a brombutyl rubber, preferably wherein the closure is a siliconized closure comprising a brombutyl rubber.

In one embodiment it might be preferred that the barrel comprises a polymer, preferably COC and/or COP; and that the closure comprises a brombutyl rubber, preferably wherein the closure is a siliconized closure comprising a brombutyl rubber.

It has been surprisingly found that the combination of a polymer syringe, preferably comprising COC and/or COP, with a polymer closure, preferably made of bromobutyl, results in increased tightness. Therefore, in one embodiment it might be preferred that a polymer syringe, preferably comprising COC and/or COP, and a polymer closure, preferably made of bromobutyl, is provided.

The object is solved by the invention according to a second aspect in that a method comprising:
Administering to a subject an effective amount of at least one pharmaceutically active ingredient comprised by a pharmaceutical composition using a system according to the first aspect of the invention is proposed.

It surprisingly turned out that after thawing the system, administering can be improved, especially made more safe, is the system is a system according to the first aspect of the invention. This is because such a system is resilient against low temperatures. In this respect reference can be made to the description provided above with respect to the first aspect of the invention.

The object is solved by the invention according to a third aspect in that a Liquid composition for use
in a method for treatment of the human or animal body by surgery or therapy, or
in a diagnostic method practiced on the human or animal body,
wherein the composition comprises at least one pharmaceutically active ingredient, and wherein the method comprises the step
administering to a subject an effective amount of said active ingredient using a system according to the first aspect of the invention is proposed.

The object is solved by the invention according to a fourth aspect in that a System according to the first aspect of the invention for use
in a method for treatment of the human or animal body by surgery or therapy, or
in a diagnostic method practiced on the human or animal body,
wherein the composition comprises at least one pharmaceutically active ingredient, and wherein the method comprises the step
administering to a subject an effective amount of said active ingredient using the system is proposed.

In one embodiment it might be preferred that for the system according to the first aspect of the invention, the method according to the second aspect of the invention, the use according to the third aspect of the invention and/or the use according to the fourth aspect of the invention, the composition comprises mRNA.

The object is solved by the invention according to a fifth aspect in that a device for producing a system according to the first aspect of the invention, the device comprising a servo motor and a laser fixedly attached, especially fixedly attached in a mechanically manner, to the servo motor, is proposed.

Such a device is particularly useful for positioning the stopper of the system by translating the stopper by means of a servo motor to a specific position within the barrel along the axial extension of the barrel.

The specific position may be determined by means of a positioning system comprising the laser.

Thus, the laser might be also comprised by a positioning system.

The object is solved by the invention according to a sixth aspect in that a method for producing a system according to the first aspect of the invention, the method comprising (i) using a device according to the fifth aspect of the invention and/or (ii) (a) providing a barrel, wherein preferably one end of the barrel is an open end and/or one further end of the barrel is a closed end, (b) filling the barrel with a liquid pharmaceutical composition, (c) providing a stopper, and/or (d) positioning the stopper by translating the stopper by means of a servo motor, especially the server motor comprised by the device, to a specific position within the barrel along the axial extension of the barrel, wherein the specific position is determined by means of a positioning system comprising a laser which laser is fixedly attached, especially fixedly attached in a mechanically manner, to the servo motor, is proposed.

The object is solved by the invention according to a seventh aspect in that a use of a system according to the first aspect of the invention for the long time storage at low temperature, is proposed.

In one embodiment it might be preferred that long time storage is 5 days or more, preferably 1 month or more, preferably 3 month or more, preferably 6 month or more, preferably, 1 year or more.

In one embodiment it might be preferred that low temperature is -20 °C or less, preferably -50 °C or less, preferably -80 °C or less.

The object is solved by the invention according to an eighth aspect in that a bundle comprising 5 or more, preferably 10 or more, preferably 30 or more, preferably 50 or more, preferably 100 or more, systems according to the first aspect of the invention, wherein for each two of the systems, the volumes of the systems' headspaces, such as the systems' headspaces A and/or the systems' headspaces B, differ by not more than 0.5 ml, preferably by not more than 0.4 ml, preferably by not more than 0.3 ml, preferably by not more than 0.2 ml, preferably by not more than 0.1 ml, is proposed.

The object is solved by the invention according to a further aspect in that a System comprising a pharmaceutical container for storing a pharmaceutical composition at low temperatures, the container having a barrel, a stopper, which fluidally closes a first end of the barrel, and a closure, which fluidally closes a second end of the barrel.

### Further Preferred Aspects

In the following, further optional features and design options are presented.

### Pharmaceutical container

The pharmaceutical container may be selected from a syringe, a cartridge and a carpule.

The inner surface of the barrel may have a water contact angle of at least 80°. High water contact angles indicate that the inner surface is hydrophobic. If the water contact angle exceeds 90° the surface is called superhydrophobic. Preferably, the inner surface of the barrel is superhydrophobic. Its water contact angle may be as high as at least 95°, or at least 100°. In preferred embodiments, the inner surface of the barrels are not plasma treated and/or otherwise hydrophilicity-increased. A typical plasma-treated inner surface will have a water contact angle significantly below 90°.

If the inner surface is hydrophobic, micro-leakage and/or protein absorption can be reduced.

Preferably, the ratio of water contact angles between the stopper's circumferential surface θ_{C} and the barrel's inner surface θ_{I} is θ_{C}/θ_{I} >0.9. Preferably, θ_{C}/θ_{I} is from 0.9 to 2, or from >1 to 1.5. In preferred embodiments, said ratio θ_{C}/θ_{I} is >1, >1.1 or >1.2. Controlling the water contact angle improves the BLGF properties. It was found that keeping the water contact angles of stopper and inner surface of the barrel within certain ranges helps achieving the desired properties.

The water contact angle can be measured according to DIN 55660-2:2011-12, chapter 5.2.2. using a static method with a drop volume of 2 µl.

The inner surface of the barrel may have a surface energy of less than 45 mN/m, or less than 40 mN/m. Preferably, the surface energy of the inner surface is higher than the surface energy of the circumferential surface of the stopper. The surface energy can be measured indirectly by calculating the value with the Owens-Wendt-Rabel-Kaelble (OWRK) method from contact angle measurements according to DIN 55660-2:2011-12, chapter 6.2.

The pharmaceutical container may be essentially lubricant-free. "Lubricant-free" means that the amount of lubricant per container is less than 100 µm, less than 30 µg, or even less than 10 µg. The above-mentioned limits may particularly apply to silicone as a lubricant.

The pharmaceutical container and/or the barrel may be partially or entirely made of a material suitable for pharmaceutical primary packaging. Suitable material includes glass or polymers. The polymers may be amorphous polymers. Transparent polymers are preferred. Suitable polymers may be selected from the group comprising cyclic olefin copolymers (COC), cyclic olefin polymers (COP), polyethylene terephthalate (PET), polycarbonate (PC), polypropylene (PP), and methylmethacrylate acrylonitrile butadiene styrene polymer (MABS). These polymers have the advantage of low density, high transparency, low birefringence, extremely low water absorption, excellent water vapor barrier properties, high rigidity, strength and hardness, excellent biocompatibility, very good resistance to acids and alkalis, and very good melt processability.

The barrel and/or the pharmaceutical container may be made of polymer. Preferably, a polymer is chosen that has low density compared to glass, such as a density of from 0.9 to 1.2 g/cm³, preferably >1 to 1.1 g/cm³. Transport costs can be reduced if low density material is used. The density may be determined using the method described in ISO 1183-1:2013-04.

For long term storage it is preferred that a material is used for the barrel that has a water vapor permeability of less than 0.1 g*mm/m²*d, preferably less than 0.07 g*mm/m²*d or even less than 0.05 g*mm/m²*d. Water vapor permeability may be tested using the method described in ISO 15106-3:2003.

In order for the break lose force (BLF) and glide force (GF) to remain sufficiently constant over a temperature range relevant for biological active agents, such as from 4°C to 25°C, it is preferred that the coefficient of linear thermal expansion (CTE) of the material used for the barrel is within a range of from 0.3 to 0.8*10⁻⁴ K⁻¹, or from 0.4 to 0.7 *10⁻⁴ K⁻¹, or from 0.3 to 0.8*10⁻⁴ K⁻¹. In preferred embodiments the ratio of the CTE of the material of the stopper and the material of the barrel, CTE_{S}/CTE_{B}, is less than 7, preferably less than 6 or at most 5. If this ratio is too high, the stopper will contract significantly, when cooling the container to e.g. 4°C in a refrigerator. This might cause leakage.

The invention offers a large freedom of design because the effects of the invention may even be achieved by uncoated barrels. Hence, the inner surface of the barrel may be uncoated.

The barrel has an inner diameter ID measured perpendicular to the container's longitudinal axis. The inner diameter ID may range from 3 mm to 40 mm, preferably from 4 mm to 20 mm. The inner diameter will generally be larger for larger barrel volumes. Larger diameters often correspond to greater BLF and GF values because of increased contact areas of the stopper's circumferential surface and the barrel's inner surface.

The wall of the barrel may be made of a transparent material. The transparent material may have a minimum transmission of at least 60% within a wavelength interval of at least 100 nm width within the wavelength range of 400 to 700 nm, measured at a thickness of the material of 1 mm. Preferably, the minimum transmission is at least 70%.

The material of the barrel wall may have a refractive index of from 1.5 to 1.6 and/or a diffraction characterized by an Abbe number of from 50 to 60. Using a material with adequate refractive index is useful to allow for good visual inspection of the contents of the pharmaceutical composition. The pharmaceutical container of this invention is suitable for administering parenteral drug compositions so that visual inspection of the container for impurities, precipitation, crystallization, and particles is of utmost importance.

The wall thickness of the barrel may be from 0.7 mm to 2.5 mm or from 1 mm to 2 mm or from 1.3 mm to 1.9 mm.

### Stopper

The stopper has a body having at least one annular protrusion, and a circumferential surface. The "circumferential surface" is the surface of the stopper that faces towards the inner surface of the barrel when the stopper is disposed in the barrel. The circumferential surface includes the surface of annular protrusions. If the stopper is coated, the surface of the coating that faces the inner surface of the barrel is part of or constitutes the circumferential surface. The "contact surface" is the part of the circumferential surface that touches the inner surface of the barrel when the stopper is inserted in the barrel.

An "annular protrusion" is a portion of the stopper that has a greater than average diameter, measured perpendicular to the longitudinal axis of the barrel. The annular protrusions touch the inner surface of the barrel so as to seal the junction between stopper and barrel. Any portion of the stopper having a greater than average diameter, but not touching the inner surface of the barrel to an extent of at least 80%, 90%, 99.9% or 100% during movement of the stopper in distal direction is not considered an "annular protrusion". Annular protrusions help keeping the stopper in the intended position within the barrel, stabilize its orientation in the proximal-distal direction, and thereby influence the BLF and GF values of the container. Further, the annular protrusions seal the junction between stopper and inner surface of the barrel.

The stopper may optionally feature one or more trailing ribs. A "trailing rib" is a portion of the stopper that has a greater than average diameter, measured perpendicular to the longitudinal axis of the barrel. However, the trailing rib has a smaller diameter than an annular protrusion so that it does not touch the barrel's inner surface to a significant extent, when the stopper is moved in the proximal-distal direction. Such trailing ribs may serve the purpose of stabilizing the stoppers orientation within the barrel, without effectively sealing the junction between stopper and inner surface. Trailing ribs do usually not significantly influence BLF and GF because their contact with the inner surface is limited, if any.

The stopper may be coated with a coating. The coating may be a polymer. In an embodiment, the coating comprises a resin, such as a fluorinated polymer such as a polymer selected from the group consisting of polytetrafluoroethylene (PTFE), densified expanded polytetrafluoroethylene (ePTFE), tetrafluoroethylene (TFE), tetrafluoroethylene-perfluoroethylene copolymer, tetrafluoroethylene-hexafluoropropylene copolymer, tetrafluoroethylene-ethylene copolymer, trichlorotrifluoroethylene, poly-vinylidene fluoride, polyvinyl fluoride, perfluoropropylvinylether, perfluoroalkoxy polymers, as well as copolymers, blends and combinations thereof. The coating may also be formed by layers comprising polyethylene, polypropylene, polyparaxylxylene, polylactic acid, as well as copolymers, blends and combinations thereof. A PTFE coating is a preferred coating option. These coatings reduce the coefficient of friction of the stopper's circumferential surface on the inner surface of the barrel. In embodiments, at least the parts of the stopper's circumferential surface that are supposed to be in contact with the barrel's inner surface will be coated.

The stopper may have an elastomeric body with an at least 10 MPa yield stress measured according ISO 527-2:2012(E) and/or a low coefficient of sliding friction below 0.23 against steel measured according to DIN EN ISO 8295/2004-10. The stopper may be made of thermoplastic elastomers and/or rubbers, such as natural or synthetic rubbers. Suitable rubber materials may be selected from the group consisting of butyl rubbers, halogenated butyl rubbers, acrylonitrilebutadiene rubbers, isoprene rubbers, neoprene rubbers, butadiene rubbers, styrene-butadiene rubbers, ethylene-propylene rubbers, isoprene-isobutylene rubbers, nitrile rubbers, and combinations and mixtures thereof. Particularly, the body of the stopper may be made of the above-listed rubbers and/or thermoplastic elastomers.

The body may be coated with a resin as described above. The coating may have a thickness of less than 1 mm, particularly from 0.5 µm to 200 µm, particularly from 10 µm to 125 µm, or from 30 to 100 µm. These thicknesses have been proven to be easily applied and sufficient for the desired effect on the friction.

The circumferential surface of the stopper may have a water contact angle of at least 100°, or even at least 110°. The circumferential surface of the stopper may be superhydrophobic. Using superhydrophobic stoppers in the containers of this invention contributes to the beneficial BLGF values due to the low coefficient of sliding friction in combination of a low adhesion disposition.

The pharmaceutical containers of this invention allow for excellent sealing between annular protrusions and inner surface of the barrel even at comparatively low compressions of the stopper. The stopper compression (SC) can be calculated as follows. SC = (OD-ID)/OD, with OD denoting the stopper's outer diameter and ID denoting the barrel's inner diameter. The stopper compression may be less than 0.1, less than 0.075, or even less than 0.05. Using a low stopper compression allows for easy gliding of the stopper within the barrel thereby keeping the total glide force variation (TGFV) very low, and reducing BLF.

The circumferential surface of the stopper and the inner surface of the barrel may at least partially contact each other in a contact area. The contact area is sometimes also referred to as the sealing area. In embodiments, the contact area will be at least 8 mm² and at most 48 mm². The contact area may be 8-48 mm² or 10-40 mm², 15-30 mm², 16-24 mm². In the case of plural annular protrusions each protrusion contributes to the contact area. A minimum contact area will be useful to achieve sufficient sealing. If the contact area is too high, BLGF values may increase too much.

### Liquid composition

The invention also relates to liquid compositions for use in a method for treatment of the human or animal body by surgery or therapy, and/or for use in a diagnostic method practiced on the human or animal body. Such a liquid composition may also be comprised by the proposed system. The liquid composition may be liquid and/or sterile. The pharmaceutical container may contain the liquid composition within the barrel.

The composition comprises at least one pharmaceutically active ingredient. "Pharmaceutically active ingredients" include therapeutic and/or diagnostic active ingredients.

The method includes administering to a subject an effective amount of said pharmaceutically active ingredient using the pharmaceutical container of this invention.

The pharmaceutically active ingredient may be a peptide or protein, such as an antibody, an enzyme, a vaccine, a receptor or the like. The pharmaceutical container of this invention is particularly suitable for administering biological active ingredients, such as peptides or proteins, since the container is very tolerant to temperature changes in terms of BLGF. This means that the BLGF does not vary significantly within a temperature range of from 4°C to 25°C. This is relevant because biological agents will usually be stored in a refrigerator so as to increase the shelf life of the product.

In embodiments, the pharmaceutically active ingredient is or comprises mRNA.

The pharmaceutical container may be combined with an injection device. The injection device may be attached to the container at the containers distal opening. The injection device may be a needle. The pharmaceutical container may be part of an auto-injector.

### Annular protrusions

The stopper may have at least two annular protrusions. In an embodiment, the stopper has from one to five annular protrusions, such as from two to four annular protrusion. In specific embodiments, the stopper may have one, two, three, four or five annular protrusions. Annular protrusions are useful for closing the juncture between inner surface of the barrel and the stopper's circumferential surface. The stopper's diameter may be greater at the annular protrusions than the average diameter of the stopper. The annular protrusions may contact the inner surface of the barrel when the stopper is moved in distal direction, e.g. when the stopper is used to push the contents of the pharmaceutical container out of the container. The surfaces of the annular protrusions may form part of the circumferential surface of the stopper.

At least one and preferably all of the annular protrusions may have a diameter that exceeds the inner diameter of the barrel. Preferably, the diameter of at least one and preferably all of the annular protrusions exceed the inner diameter of the barrel by at least 0.05 mm, or at least 0.1 mm, or at least 0.15 mm. The outer diameter of the annular protrusion may be equivalent to the outer diameter of the stopper. The diameter is measured perpendicular to the barrel's longitudinal axis.

### Surface roughness

Surface roughness values can for example be controlled by adjusting the temperature during injection molding. In embodiments, the inner surface of the barrel may have a surface roughness Ra of less than 100 nm. The surface roughness Ra indicated herein may be an average, or a maximum surface roughness. Preferably, the surface roughness Ra of the inner surface of the barrel is less than 80 nm, less than 70 nm, less than 60 nm, less than 50 nm or less than 40 nm. The surface roughness Ra may be at least 1 nm, at least 3 nm or at least 7 nm. Preferred ranges include surface roughness Ra values from 1 nm to 80 nm, from 3 nm to 70 nm, or from 7 nm to 50 nm.

Surface roughness can additionally or alternatively be given as Rms roughness. In embodiments, the inner surface of the barrel may have a surface roughness Rms of less than 150 nm. The surface roughness Rms indicated herein may be an average, or a maximum surface roughness. Preferably, the surface roughness Rms of the inner surface of the barrel is less than 120 nm, less than 100 nm, less than 80 nm, less than 70 nm or less than 60 nm. The surface roughness Rms may be at least 2 nm, at least 5 nm or at least 8 nm. Preferred ranges include surface roughness Ra values from 2 nm to 120 nm, from 5 nm to 100 nm, or from 8 nm to 60 nm. Surface roughness influences the stopper's ability move while contacting the barrel's inner surface. For example, the coefficient of friction may be very high, if surface roughness is very high.

The surface roughness of the inner surface of the barrel may decline from the stopper's start position to its end position by at least 3 % Ra and/or Rms relative to the roughness value at the start position. In preferred embodiments the surface roughness Ra declines from start to end position of the stopper by at least 5 %, at least 7 %, at least 10 %, at least 20 %, at least 30 %, at least 40 %, at least 50 %, at least 60 % or at least 70 %. The surface roughness Rms may decline from start to end position of the stopper by at least 5 %, at least 7 %, at least 10 %, at least 20 %, at least 30 %, at least 40 %, at least 50 %, at least 60 % or at least 70 %.

The stopper's start position is the position where the stopper is located within the barrel before the container is used for drug delivery. In embodiments, the pharmaceutical container is a prefilled syringe (especially in case of the proposed system). In a pre-filled syringe the start position is the location of the stopper before use. This will usually be the most proximal position of the stopper. The stopper's end position is the position where the stopper is located after pushing the nominal volume of the container out of the barrel, e.g. when the stopper touches the distal end of the barrel. The stopper's start position may be located within a distance of up to 20% of the container's length from its proximal end. The end position may be located within a distance of from 80% to 100% of the container's length from its proximal end.

The inner surface of the barrel may have a surface roughness distribution such that the surface roughness measured at the start position SP, a middle position MP, and an end position EP is as follows, wherein the middle position may be located half way between start and end position:
SP 100%
MP 40 to 60%
EP 20 to 35%

Controlling the surface roughness may contribute to a very low TGFV. Surface roughness can be controlled by adjusting production parameters like melt temperatures, molding times and polymer blends, or by surface treatment like coating or plasma treatment. The pharmaceutical containers may be made by injection molding. Injection molding requires the container to be at least slightly conical, i.e. the inner diameter of the barrel will decrease from start to end position. Thus, compression of the stopper increases from start to end position so that GFₘₐₓ would increase and TGFV will increase as well. Within this description any reference to the inner diameter of the barrel means the maximum inner diameter of the barrel, unless otherwise indicated.

The described surface roughness distribution may be achieved for example by positioning the injection nozzle closer to the end position than to the start position during injection molding so that the polymer melt temperature is higher at the end position than at the start position during injection. Or the mold temperature may be influenced by segmental heating and/or cooling to create a temperature gradient in the barrel direction.

The surface roughness values can be measured using a white light interferometer according to DIN EN ISO 25178-2:2012, DIN EN ISO 25178-6:2010 and DIN EN ISO 25178-604:2013-12 (together with DIN EN ISO 4288:1998 and DIN EN ISO 3274:1998).

### Break loose and glide force

The pharmaceutical containers of this invention may exhibit a maximum BLGF of not more than 15 N during a BLGF test. In preferred embodiments, the maximum BLGF may be limited to 13N, 10 N, 9 N, 8 N, 7 N, 6 N, 5 N or even 4 N. The BLF may be at least 0.1 N or at least 0.5 N so as to avoid any unintended movement of the stopper.

In embodiments, the maximum BLGF may correlate to the inner diameter of the barrel. Preferably, the ratio of maximum BLGF and the barrel's inner diameter ID is BLGF/ID <1 N/mm. Preferably BLGF/ID may be at least 0.5 N/mm, or at least 0.6 N/mm. In embodiments, BLGF/ID may be limited to ≤0.95 N/mm, ≤0.9 N/mm, ≤0.85 N/mm or ≤0.8 N/mm.

The container exhibits a ratio of the BLF relative to the GF of BLF/GF ≤ 2 during a BLGF test. Preferably, the ratio of the BLF relative to the GF is characterized by BLF/GF ≤ 3 even after accelerated aging for 105 days. In preferred embodiments, the ratio BLF/GF is <2, <1.8, <1.7, or even <1.5 for the containers of this invention. In preferred embodiments, the ratio BLF/GF may be <2, <1.8, <1.7, or even <1.5 for the containers of this invention after accelerated aging for 105 days. Preferably, the relative difference in the ratios BLF/GF of aged containers (accelerated aging 105d), and non-aged containers (BLF/GF_{105d} - BLF/GF_{0d})/ BLF/GF_{105d} is less than 10%, preferably less than 5%.

The total glide force variation TGFV = GFₘₐₓ - GFₘᵢₙ measured when the stopper is moved from start position to its end position may be TGFV < 2 N, <1.8 N or even <1.6 N. Preferably, the relative difference in the TGFV of aged containers (accelerated aging 105d), and non-aged containers (TGFV_{105d} - TGFV_{0d})/ TGFV_{105d} is less than 40%, preferably less than 35%.

The mean values of BLF and GF may be calculated using at least 12 containers, preferably at least 15 containers. The mean BLF of the pharmaceutical containers of this invention may be <9 N, <8 N, <7 N, <6 N, <5 N, <4 N, <3 N, or even <2 N. The mean GF of the pharmaceutical containers of this invention may be <9 N, <8 N, <7 N, <6 N, <5 N, <4 N, <3 N, or even <2 N. Preferably, the relative difference in the BLF of aged containers (accelerated aging 105d), and non-aged containers (BLF_{105d} - BLF_{0d})/ BLF_{105d} is less than 25%, <20%, <15%, <10% or even <5%. The relative difference in the GF of aged containers (accelerated aging 105d), and non-aged containers (GF_{105d} - GF_{0d})/ GF_{105d} is less than 25%, <20%, <15%, <10% or even <5%.

Keeping the BLF and GF values within the ranges of this invention contributes to a sufficiently constant elution of liquid composition from the container during application. Particularly, if the BLF is much higher than the GF a large bolus may be eluted when the stopper breaks loose from the inner surface of the barrel. Also, if the GF is not sufficiently constant, the elution rate of liquid composition may vary.

"Accelerated aging" refers to an aging process where the respective containers are stored at 40°C and 75% relative humidity. For example, some containers may be stored at these conditions for 105 days for comparison. Accelerated aging can be performed to estimate the influence of aging on the properties of the pharmaceutical containers of this invention.

### Ratios

The container may exhibit a ratio of the break loose force (BLF) relative to the glide force (GF) of BLF/GF ≤ 2 during a break loose and glide force (BLGF) test. It was found that controlling the ratio of BLF and GF is important because if the BLF is too high compared to GF, the user of the container will have to push very hard to remove the stopper from its start position so that the stopper might be pushed too fast all the way to the end position after the stopper breaks loose. Keeping the ratio BLF/GF in a balanced range, will facilitate a controlled drug delivery without unnecessary pain for the patient. Also, the risk of leakage of the contents of the container will be smaller, if the acceleration of the stopper is limited after breaking loose. The ratio BLF/GF may be >1. It is an aspect of this invention to keep the BLF/GF ratio essentially constant even after storage of the container during administration.

The total glide force variation TGFV = GFₘₐₓ - GFₘᵢₙ measured when the stopper is moved from start position to its end position may preferably be TGFV < 2 N. It is important to control TGFV because the difference between maximum GF and minimum GF will strongly influence the user's ability to dose the drug composition stored in the container adequately.

Preferably, here the BLF and GF at room temperature is used.

### Break loose and glide force test

The BLGF test is conducted on a universal testing machine at room temperature, e.g. 293.15 K, 20 °C or 23 °C. A BLGF testing device with a 50 N test cup is used for this purpose. The samples were fixed in vertical orientation in a universal testing machine model 106, 2 kN from TesT AG, CH-6331 Hünenberg, Switzerland.

For this test plungers with flat ends, i.e. without any threads are used.

The BLF is the force needed to move the stopper from its original position. The GF is the force needed to keep the plunger moving after breaking it loose.

The pharmaceutical containers are filled with water for injection. After filling the specimen they are either stored or tested immediately, depending on the test purpose. The specimen are tested without needles.

The specimen are inserted into the holder and the pressure stamp is moved towards the plunger at a rate of 20 mm/min. Once a force of 0.25 N is measured the machine switches to the test rate of 100 mm/min and starts recording the data. The experiment ends when the measured force exceeds 35 N, which is usually the case when the distal end of the barrel is reached.

The BLF is the highest force measured within the first 4 mm of stopper movement. The mean and maximum GF values are measured within a test range starting after 4 mm of movement and ending 10 mm before reaching the distal end of the barrel.

### Break loose force to glide force ratio

The container may exhibit a ratio of the break loose force (BLF) relative to the glide force (GF) of BLF/GF ≤ 2 during a break loose and glide force (BLGF) test. It was found that controlling the ratio of BLF and GF is important because if the BLF is too high compared to GF, the user of the container will have to push very hard to remove the stopper from its start position so that the stopper might be pushed too fast all the way to the end position after the stopper breaks loose. Keeping the ratio BLF/GF in a balanced range, will facilitate a controlled drug delivery without unnecessary pain for the patient. Also, the risk of leakage of the contents of the container will be smaller, if the acceleration of the stopper is limited after breaking loose. The ratio BLF/GF may be >1. It is an aspect of this invention to keep the BLF/GF ratio essentially constant even after storage of the container during administration.

The total glide force variation TGFV = GFₘₐₓ - GFₘᵢₙ measured when the stopper is moved from start position to its end position may preferably be TGFV < 2 N. It is important to control TGFV because the difference between maximum GF and minimum GF will strongly influence the user's ability to dose the drug composition stored in the container adequately.

The BLF, the GF and the BLGF can be measured according to the method described herein as the BLGF test.

### The angles X, A and their ratio X/A

The rising edge of the most proximal annular protrusion and the inner surface of the barrel span angle X which opens in the distal direction, the falling edge of the most distal annular protrusion and the inner surface span an angle A opening in the proximal direction. A "rising edge" in the context of this invention is an edge of an annular protrusion that extends in the direction of the inner surface of the barrel, when the stopper is inserted in the barrel, following the circumferential surface of the stopper in proximal-distal direction. A "falling edge" is an edge of an annular protrusion that extends in the direction towards the central longitudinal axis of the barrel, when the stopper is inserted in the barrel, following the circumferential surface of the stopper in proximal-distal direction.

In preferred embodiments, the ratio X/A is from >1.1 to 1.7. If the ratio of X/A is at least 1.05, the BLGF values are improved. In preferred embodiments the ratio X/A is at least 1.1, at least 1.15, at least 1.2 or at least 1.25. The ratio may preferably be limited to up to 1.7, up to 1.65, up to 1.6, up to 1.55, up to 1.5, or up to 1.45.

The angles X and/or A may be from >90° to <180°. Preferably A is from 130° to 170°. The minimum value of A may be at least 100°, at least 110°, at least 120° or at least 130°. The upper limit of A may be 170°, 160°, 150° or 140°. Preferably X is from 131° to 175°. The minimum value of X may be at least 101°, at least 111°, at least 121° or at least 131°. The upper limit of X may be 170°, 160°, 150° or 140°.

Keeping the angles and their ratio within appropriate ranges will contribute to solving the problem underlying this invention. Particularly, if angle X is too small, the ratio BLF/GF will increase. With the related angle ratio, the force inserted by a plunger rod needs to be distributed and shared inside the plunger towards the sealing lips in a uniform and controlled way, which can be imagined by strength lines and the uncontrolled deformation of the plunger can be avoided by the mentioned ratio of the design angles.

### Directions

The proximal-distal direction is equivalent to the direction of a vector pointing from the proximal end to the distal end. The rising edge of the most proximal annular protrusion and the inner surface of the barrel span angle X in the distal direction, the falling edge of the most distal annular protrusion and the inner surface span an angle A in the proximal direction, and the ratio X/A may preferably be at least 1.05.

### Further options

The container may be selected from a syringe, a cartridge and a carpule.

The inner surface of the barrel may have a surface roughness Ra of less than 100 nm, a surface roughness Rms of less than 150 nm, a surface energy of less than 45 mN/m, and/or a silicone content of less than 100 µg, or less than 30 µg or less than 1 µg per barrel.

The inner surface of the barrel may have a water contact angle of at least 80°, preferably at least 85°.

The stopper may have a proximal end suitable for contacting a plunger rod, and a distal end suitable for contacting a pharmaceutical composition.

The pharmaceutical container may further comprise a plunger rod connected to the proximal end of the stopper.

The stopper may have one or more, preferably at least two, annular protrusions contacting the inner surface of the barrel when the stopper moves in distal direction.

The annular protrusions each may have a rising edge and a falling edge in proximal-distal direction.

The rising edge of the most proximal annular protrusion and the inner surface of the barrel may span angle X in the distal direction, and/or the falling edge of the most distal annular protrusion and the inner surface may span an angle A in the proximal direction, wherein preferably the ratio X/A is at least 1.05. Preferably the ratio X/A is from >1.1 to 1.7, and/or A is from 130° to 170°.

Preferably, throughout the application, room temperature is 293 K (293.15 K), which is 20 °C (20.00 °C).

If not stated otherwise, the dimensions (volume, distance) is the dimension at room temperature (20°C).

### Preferred Embodiments

The embodiments according to the following examples are particularly preferred:
Example 1. System for storing a pharmaceutical composition at low temperatures, the system comprising
   - a pharmaceutical container for storing a pharmaceutical composition at low temperatures, the container having a barrel, a stopper, which fluidally closes a first end of the barrel, and a closure, which fluidally closes a second end of the barrel;
   - a liquid pharmaceutical composition and a gas, both, arranged within the barrel of the container between the stopper and the closure.
Example 2. System, especially according to Example 1, for storing a pharmaceutical composition at low temperatures, the system comprising
   - a pharmaceutical container for storing a pharmaceutical composition at low temperatures, the container having a barrel, a stopper, which fluidally closes a first end of the barrel, and a closure, which fluidally closes a second end of the barrel;
   - a liquid pharmaceutical composition and a gas, both, arranged within the barrel of the container between the stopper and the closure;
   wherein a volume enclosed by the barrel which is occupied by the gas is defined as headspace of the system.
Example 3. System, especially according to any one of the preceding Examples, for storing a pharmaceutical composition at low temperatures, the system comprising
   - a pharmaceutical container for storing a pharmaceutical composition at low temperatures, the container having a barrel, a stopper, which fluidally closes a first end of the barrel, and a closure, which fluidally closes a second end of the barrel;
   - a liquid pharmaceutical composition and a gas, both, arranged within the barrel of the container between the stopper and the closure;
   wherein the liquid pharmaceutical composition comprises an additive.
Example 4. System , especially according to any one of the preceding Examples, for storing a pharmaceutical composition at low temperatures, the system comprising
   - a pharmaceutical container for storing a pharmaceutical composition at low temperatures, the container having a barrel, a stopper, which fluidally closes a first end of the barrel, and a closure, which fluidally closes a second end of the barrel;
   - a liquid pharmaceutical composition and a gas, both, arranged within the barrel of the container between the stopper and the closure,

   wherein, for a vertical orientation of the container with the first end being at the bottom and the second end being at the top, the volume enclosed by the barrel between the stopper and the closure which is occupied by the gas is defined as headspace of the system,
   and wherein, at room temperature, the volume occupied by the headspace is 1 % or more of the volume occupied by the composition.
Example 5. System according to any one of the examples 1 to 3, wherein, especially for a particular orientation of the container, a volume enclosed by the barrel which is occupied by the gas is defined as headspace of the system.
Example 6. System according to example 5, wherein, for a vertical orientation of the container with the first end being at the bottom and the second end being at the top, the volume enclosed by the barrel between the stopper and the closure which is occupied by the gas is defined as headspace A of the system.
Example 7. System according to example 4 or 5, wherein, for a vertical orientation of the container with the second end being at the bottom and the first end being at the top, the volume enclosed by the barrel between the stopper and the closure which is occupied by the gas is defined as headspace B of the system.
Example 8. System according to any one of the examples 1 to 3 and 5 to 7, wherein, at room temperature, the volume occupied by the headspace, such as the headspace A and/or the headspace B, is 1 % or more of the volume occupied by the composition.
Example 9. System according to any one of the preceding examples, wherein
   the headspace B has a cylindrical volume domain portion which volume domain portion has a specific diameter equal to the inside diameter of the barrel and a specific height, which specific height preferably is measured from the central point of the stopper to the surface of the liquid pharmaceutical composition facing the stopper, wherein the specific height has a value of 0.1 mm or more.
Example 10. System according to example 9, wherein the specific height is
   (i) 0.2 mm or more, preferably 0.5 mm or more, preferably 0.7 mm or more, preferably 1 mm or more, preferably between 2 mm or more, preferably 3 mm or more, preferably 4 mm or more, preferably 5 mm or more, preferably 6 mm or more, preferably 7 mm or more, preferably 8 mm or more, preferably 9 mm or more, preferably 10 mm or more, preferably 11 mm or more, preferably 12 mm or more, preferably 13 mm or more, preferably 14 mm or more, preferably 15 mm or more,
   (ii) 15 mm or less, preferably 14 mm or less, preferably 13 mm or less, preferably 12 mm or less, preferably 11 mm or less, 10 mm or less, preferably 9 mm or less, preferably 8 mm or less, preferably 7 mm or less, preferably 6 mm or less, preferably 5 mm or less, preferably 4 mm or less, preferably 3 mm or less, preferably 2 mm or less,
      and/or
   (iii) between 1 mm and 15 mm, preferably between 1 mm and 10 mm, preferably between 1 mm and 8 mm, preferably between 1 mm and 3 mm, such as 2 mm, or between 3 mm and 5 mm , such as 4 mm, or between 5 mm and 7 mm, such as 6 mm, or between 7 mm and 9 mm, such as 8 mm.
Example 11. System according to example 9 or 10, wherein
   the ratio [mm/mm] of the inside diameter of the barrel and the specific height is
   (i) 0.3 or more, preferably 0.7 or more, preferably 1 or more, preferably 1.05 or more, preferably 1.1 or more, preferably 1.3 or more, preferably 1.5 or more, preferably 2 or more, preferably 2.5 or more, preferably 3 or more, preferably 3.5 or more, preferably 4 or more, preferably 4.5 or more, preferably 5 or more, preferably 7 or more, preferably 10 or more,
   (ii) 10 or less, preferably 6 or less, preferably 5 or less, preferably 4 or less, preferably 3 or less, preferably 2 or less, preferably 1.5 or less, preferably 1.3 or less, preferably 1.0 or less, preferably 0.7 or less, preferably 0.5 or less,
      and/or
   (iii) between 0.3 and 10, preferably between 1 and 10, preferably between 1 and 5, preferably between 1 and 2 or between 2 and 4 or between 3 and 5.
Example 12. System according to any one of the preceding examples, wherein
   the total length of the barrel, especially measured along the axial extension of the barrel, is
   (i) 40 mm or more, preferably 45 mm or more, preferably 50 mm or more, preferably 55 mm or more, preferably 60 mm or more, preferably 65 mm or more, preferably 70 mm or more, preferably 75 mm or more, preferably 80 mm or more,
   (ii) 80 mm or less, preferably 75 mm or less, preferably 70 mm or less, preferably 65 mm or less, preferably 60 mm or less, preferably 55 mm or less, preferably 50 mm or less, preferably 45 mm or less, preferably 40 mm or less,
      and/or
   (iii) between 40 mm and 80 mm, preferably between 40 mm and 60 mm, especially between 45 mm and 55 mm, particularly between 45 mm and 50 mm, or between 60 mm and 70 mm, particularly between 63 mm and 67 mm.
Example 13. System according to any one of the preceding examples, wherein
   the inside diameter of the barrel is
   (i) 5 mm or more, preferably 6 mm or more, preferably 7 mm or more, preferably 8 mm or more, preferably 9 mm or more,
   (ii) 10 mm or less, preferably 9 mm or less, preferably 8 mm or less, preferably 7 mm or less, preferably 6 mm or less, preferably 5 mm or less,
      and/or
   (iii) between 5 mm and 10 mm, preferably between 5 mm and 7 mm, especially between 6 mm and 7 mm, particularly between 6 mm and 6.5 mm such as 6.35 mm, or between 7 mm and 9 mm, especially between 8 mm and 9 mm, particularly between 8.5 mm and 9 mm such as 8.65 mm.
Example 14. System according to any one of the preceding examples, wherein
   (i) the total length of the barrel, especially measured along the axial extension of the barrel, is between 45 mm and 50 mm and the inside diameter of the barrel is between 8 mm and 9 mm,
      or
   (ii) the total length of the barrel, especially measured along the axial extension of the barrel, is between 60 mm and 70 mm and the inside diameter of the barrel is between 5 mm and 7 mm.
Example 15. System according to any one of the preceding examples, wherein
   the following equation(s) is/are fulfilled:
   (X/Y)/Z≤V; and/or
   W≤(X/Y)/Z;
   wherein X is the volume (ml) of the pharmaceutical composition at room temperature;
   wherein Y is the volume (ml) of the headspace at room temperature;
   wherein Z is the inner diameter (mm) of the barrel;
   wherein V (1/mm) is 1.5, preferably 1.4, preferably 1.3, preferably 1.2, preferably 1.1, preferably 1.0, preferably 0.9, preferably 0.8, preferably 0.7, preferably 0.6, preferably 0.5, preferably 0.4, preferably 0.3, preferably 0.2, preferably 0.1;
      and/or
   wherein W (1/mm) is 0.01, preferably 0.05, preferably 0.1, preferably 0.2, preferably 0.3, preferably 0.4, preferably 0.5, preferably 0.6, preferably 0.7.
Example 16. System according to any one of the preceding examples, wherein
   the liquid pharmaceutical composition comprises an additive.
Example 17. System according to example 16, wherein
   the additive is selected from a group consisting of salt, sugar, lipid and acid.
Example 18. System according to example 16 or 17, wherein
   the additive is selected from a group consisting of NaCl, KCI, sucrose, sodium acetate, acetic acid, ((4-hydroxybutyl)azanediyl)bis(hexan-6,1-diyl)bis(2-hexyldecanoat), 2[(polyethylene glycol)-2000]-N,N-ditetradecylacetamide, 1,2-Distearoyl-sn-glycero-3-phosphocholine, SM(sphyngomyelin)-102, polyethylene glycol [PEG] 2000 dimyristoyl glycerol [DMG], 1,2-distearoyl-sn-glycero-3-phosphocholine [DSPC], cholesterol, polyethylene glycol (PEG), H3PO4, XH2PO4, X2HPO4 and X3PO4, wherein X is Na and/or K.
Example 19. System according to example 16, wherein the additive is NaCl.
Example 20. System according to example 16, wherein the additive is KCI.
Example 21. System according to example 16, wherein the additive is sucrose.
Example 22. System according to example 16, wherein the additive is sodium acetate.
Example 23. System according to example 16, wherein the additive is acetic acid.
Example 24. System according to example 16, wherein the additive is a lipid, preferably ((4-hydroxybutyl)azanediyl)bis(hexan-6,1-diyl)bis(2-hexyldecanoat), 2[(polyethylene glycol)-2000]-N,N-ditetradecylacetamide, 1,2-Distearoyl-sn-glycero-3-phosphocholine, SM(sphyngomyelin)-102, polyethylene glycol [PEG] 2000 dimyristoyl glycerol [DMG], 1,2-distearoyl-sn-glycero-3-phosphocholine [DSPC] and/or cholesterol.
Example 25. System according to example 16, wherein the additive is polyethylene glycol (PEG).
Example 26. System according to example 16, wherein the additive is H3PO4.
Example 27. System according to example 16, wherein the additive is XH2PO4, wherein X is Na and/or K.
Example 28. System according to example 16, wherein the additive is X2HPO4, wherein X is Na and/or K.
Example 29. System according to example 16, wherein the additive is X3PO4, wherein X is Na and/or K.
Example 30. System according to example 16, wherein the additive is cholesterol.
Example 31. System according to any one of examples 16 to 30, wherein the concentration of the additive is:
   (i) 0.01 mol/l or more, preferably 0.05 mol/l or more, preferably 0.1 mol/l or more, preferably 0.2 mol/l or more, preferably 0.3 mol/l or more, preferably 0.4 mol/l or more, preferably 0.5 mol/l or more, preferably 0.7 mol/l or more, preferably 1 mol/l or more,
   (ii) 1 mol/l or less, preferably 0.9 mol/l or less, preferably 0.8 mol/l or less, preferably 0.7 mol/l or less, preferably 0.6 mol/l or less, preferably 0.5 mol/l or less, preferably 0.4 mol/l or less, preferably 0.3 mol/l or less, preferably 0.2 mol/l or less,
      and/or
   (iii) between 0.1 mol/l and 1 mol/l, preferably between 0.1 mol/l and 0.8 mol/l, preferably between 0.1 mol/l and 0.5 mol/l, preferably between 0.2 mol/l and 0.4 mol/l, preferably 0.25 mol/l.
Example 32. System according to any one of examples 16 to 31, wherein the concentration of the additive is:
   (i) 1 g/l or more, preferably 2g/l or more, preferably 5 g/l or more, preferably 10 g/l or more, preferably 20 g/l or more, preferably 30 g/l or more, preferably 40 g/l or more, preferably 50 g/l or more, preferably 75 g/l or more, preferably 100 g/l or more, preferably 150 g/l or more, preferably 200 g/l or more,
   (ii) 300 g/l or less, preferably 200 g/l or less, preferably 150 g/l or less, preferably 120 g/l or less, preferably 100 g/l or less, preferably 90 g/l or less, preferably 80 g/l or less, preferably 70 g/l or less, preferably 60 g/l or less, preferably 50 g/l or less, preferably 40 g/l or less, preferably 30 g/l or less, preferably 20 g/l or less, preferably 10 g/l or less, preferably 5 g/l or less,
      and/or
   (iv) between 1 g/l and 300 g/l, preferably between 5 g/l and 200 g/l, preferably between 20 g/l and 150 g/l, preferably between 50 g/l and 120 g/l, preferably between 60 g/l and 100 g/l, preferably between 70 g/l and 90 g/l.
Example 33. System according to any one of the preceding examples, wherein the concentration of all compounds in the pharmaceutical composition is
   (i) 0.01 mol/l or more, preferably 0.05 mol/l or more, preferably 0.1 mol/l or more, preferably 0.2 mol/l or more, preferably 0.3 mol/l or more, preferably 0.4 mol/l or more, preferably 0.5 mol/l or more, preferably 0.7 mol/l or more, preferably 1 mol/l or more,
   (ii) 1 mol/l or less, preferably 0.9 mol/l or less, preferably 0.8 mol/l or less, preferably 0.7 mol/l or less, preferably 0.6 mol/l or less, preferably 0.5 mol/l or less, preferably 0.4 mol/l or less, preferably 0.3 mol/l or less, preferably 0.2 mol/l or less,
      and/or
   (iii) between 0.1 mol/l and 1 mol/l, preferably between 0.1 mol/l and 0.8 mol/l, preferably between 0.1 mol/l and 0.5 mol/l, preferably between 0.2 mol/l and 0.4 mol/l, preferably 0.25 mol/l.
Example 34. System according to any one of the preceding examples, wherein, at room temperature, the headspace, such as the headspace A and/or the headspace B, is a volume which is
   (i) 1.3 % or more, preferably 1.5 % or more, preferably 1.7 % or more, preferably 2 % or more, preferably 2.5 % or more, preferably 3 % or more, preferably 3.5 % or more, preferably 4 % or more, preferably 4.5 % or more, preferably 5 % or more, preferably 6 % or more, preferably 7 % or more, preferably 8 % or more, preferably 9% or more, preferably 10 % or more, preferably 11 % or more, preferably 12 % or more, preferably 13 % or more, preferably 14 % or more, preferably 15 % or more, preferably 17 % or more, preferably 20 % or more,
   (ii) 20 % or less, preferably 17 % or less, preferably 15 % or less, preferably 14 % or less, preferably 13 % or less, preferably 12 % or less, preferably 11 % or less, preferably 10 % or less, preferably 9 % or less, preferably 8 % or less, preferably 7 % or less, preferably 6 % or less, preferably 5 % or less, preferably 4.5 % or less, preferably 4 % or less, preferably 3.5 % or less, preferably 3 % or less, preferably 2.5 % or less, preferably 2 % or less, preferably 1.7% or less, preferably 1.5 % or less, preferably 1.3 % or less,
      and/or
   (iii) between 1 % and 20 %, especially between 1.5 % and 3 %, between 2 % and 5 %, between 4 % and 8 %, between 7 % and 12 %, between 10 % and 15 %, between 12 % and 17 % and/or between 15 % and 20 %,
   respectively, of the volume occupied by the composition.
Example 35. System according to any one of the preceding examples, wherein
   (i) the stopper being slidably arranged within the barrel,
   (ii) the stopper having a circumferential surface at least partially contacting an inner surface of the barrel,
      and/or
   (iii) the first end is a proximal end of the container, especially of the barrel, and/or the second end is a distal end of the container, especially of the barrel.
Example 36. System according to any one of the preceding examples, wherein
   the system or parts thereof is/are designed in such a way, especially the headspace, such as the headspace A and/or the headspace B, at room temperature, the barrel, the stopper and/or the composition are adapted to each other in such a way, that in case of an expansion of the pharmaceutical composition, especially an expansion of the composition during cooling the system,
   preferably (a) from a temperature above 4 degrees C, especially from room temperature, and/or (b) to or below zero degrees C, especially to or below the freezing point of the composition and/or to or below a storage temperature of the system,
      (i) the pharmaceutical composition can expand or expands by displacing and/or compressing the gas, hence, reducing the volume of the headspace, such as the headspace A and/or the headspace B,
      (ii) the maximal force applied to the stopper by the pharmaceutical composition and/or the gas, especially in an axial direction, is lower than the break loose force of the stopper,
      (iii) the stopper moves 5 mm or less, preferably 4 mm or less, preferably 3.5 mm or less, preferably 3 mm or less, preferably 2.7 mm or less, preferably 2.5 mm or less, preferably 2.3 mm or less, preferably 2.0 mm or less, preferably 1.7 mm or less, preferably 1.5 mm or less, preferably 1.3 mm or less, preferably 1.0 mm or less, preferably 0.8 mm or less, preferably 0.6 mm or less, preferably 0.5 mm or less, preferably 0.4 mm or less, preferably 0.3 mm or less, preferably 0.2 mm or less, preferably 0.1 mm or less,
         and/or
      (iv) the stopper moves 0.01 mm or more, preferably 0.05 mm or more, preferably 0.1 mm or more, preferably 0.3 mm or more, preferably 0.5 mm or more, preferably 0.7 mm or more, preferably 1.0 mm or more,
   wherein preferably the barrel is oriented in such a vertical orientation during cooling that (a) the first end is at the bottom and/or the second end is at the top and/or (b) the pharmaceutical composition and the gas are arranged along the axial direction of the container, especially of the barrel.
Example 37. System according to any one of the preceding examples, wherein
   (i) the storage temperature is
      (a) -100 degrees C or above, preferably -80 degrees C or above, preferably -60 degrees C or above, preferably -40 degrees C or above, preferably -20 degrees C or above, preferably -10 degrees C or above,
      (b) 0 degrees C or below, -1 degrees C or below, preferably -5 degrees C or below, preferably -15 degrees C or below, preferably -25 degrees C or below, preferably -35 degrees C or below, preferably -55 degrees C or below, preferably -75 degrees C or below, preferably -95 degrees C or below,
         and/or
      (c) between -100 and 0 degrees C, preferably between -80 degrees C and -5 degrees C, preferably between -60 degrees C and -20 degrees C, preferably between -50 and -40 degrees C, and/or (d) 0 degrees C, -10 degrees C, -20 degrees C, -30 degrees C, -40 degrees C or -50 degrees C;
      and/or
   (ii) the storage temperature is below the freezing temperature of the composition.
Example 38. System according to any one of the preceding examples, wherein
   (i) the pharmaceutical composition comprises H2O, especially more than 80 % (w/w), preferably more than 90 % (w/w), preferably more than 95 % (w/w), preferably more than 97 % (w/w), preferably more than 98 % (w/w), of the composition is H2O;
      and/or
   (ii) the gas comprises or is air, CO2, N2, Ar and/or O2.
Example 39. System according to any one of the preceding examples, wherein
   the total length of the container, especially measured along the axial extension of the container, is
   (i) 30 mm or more, preferably 40 mm or more, preferably 50 mm or more, preferably 60 mm or more, preferably 70 mm or more, preferably 80 mm or more, preferably 90 mm or more, preferably 100 mm or more, preferably 110 mm or more, preferably 120 mm or more, preferably 130 mm or more, preferably 150 mm or more, preferably 170 mm or more, preferably 190 mm or more,
   (ii) 200 mm or less, preferably 150 mm or less, preferably 140 mm or less, preferably 130 mm or less, preferably 110 mm or less, preferably 100 mm or less, preferably 90 mm or less, preferably 80 mm or less, preferably 70 mm or less, preferably 60 mm or less, preferably 50 mm or less, preferably 40 mm or less,
      and/or
   (iii) between 30 mm and 200 mm, preferably between 50 mm and 150 mm, especially between 50 mm and 100 mm, such as between 60 mm and 90 mm and/or between 90 mm and 150 mm.
Example 40. System according to any one of the preceding examples, wherein the total length of the barrel, especially measured along the axial extension of the barrel, is
   (i) 30 mm or more, preferably 40 mm or more, preferably 50 mm or more, preferably 60 mm or more, preferably 70 mm or more, preferably 80 mm or more, preferably 90 mm or more, preferably 100 mm or more, preferably 110 mm or more, preferably 120 mm or more, preferably 130 mm or more, preferably 150 mm or more, preferably 170 mm or more, preferably 190 mm or more,
   (ii) 200 mm or less, preferably 150 mm or less, preferably 140 mm or less, preferably 130 mm or less, preferably 110 mm or less, preferably 100 mm or less, preferably 90 mm or less, preferably 80 mm or less, preferably 70 mm or less, preferably 60 mm or less, preferably 50 mm or less, preferably 40 mm or less,
      and/or
   (iii) between 30 mm and 200 mm, preferably between 40 mm and 130 mm, especially between 40 mm and 80 mm, between 50 mm and 80 mm and/or between 70 mm and 130 mm.
Example 41. System according to any one of the preceding examples, wherein
   the inside diameter of the barrel is
   (i) 5 mm or more, preferably 8 mm or more, preferably 10 mm or more, preferably 13 mm or more, preferably 15 mm or more, preferably 18 mm or more, preferably 20 mm or more, preferably 25 mm or more, preferably 30 mm or more,
   (ii) 30 mm or less, preferably 28 mm or less, preferably 25 mm or less, preferably 20 mm or less, preferably 15 mm or less, preferably 13 mm or less, preferably 10 mm or less, preferably 8 mm or less, preferably 7 mm or less, preferably 6 mm or less,
      and/or
   (iii) between 5 mm and 30 mm, preferably between 5 mm and 10 mm, between 10 mm and 15 mm, between 14 mm and 18 mm, between 17 mm and 20 mm and/or between 20 mm and 30 mm.
Example 42. System according to any one of the preceding examples, wherein
   the outside diameter of the barrel is
   (i) 5 mm or more, preferably 8 mm or more, preferably 10 mm or more, preferably 13 mm or more, preferably 15 mm or more, preferably 18 mm or more, preferably 20 mm or more, preferably 25 mm or more, preferably 30 mm or more, preferably 35 mm or more, preferably 40 mm or more,
   (ii) 40 mm or less, preferably 35 mm or less, 30 mm or less, preferably 28 mm or less, preferably 25 mm or less, preferably 20 mm or less, preferably 15 mm or less, preferably 13 mm or less, preferably 10 mm or less, preferably 7 mm or less, preferably 5 mm or less,
      and/or
   (iii) between 5 mm and 40 mm, preferably between 5 mm and 9 mm, between 8 mm and 13 mm, between 12 mm and 16 mm, between 15 mm and 20 mm, between 19 mm and 25 mm and/or between 25 mm and 35 mm.
Example 43. System according to any one of the preceding examples, wherein
   at room temperature the composition has a volume of
   (i) 0.1 ml or more, preferably 0.2 ml or more, preferably 0.3 ml or more, preferably 0.5 ml or more, preferably 0.7 ml or more, preferably 1 ml or more, preferably 1.5 ml or more, preferably 2 ml or more, preferably 3 ml or more, preferably 5 ml or more, preferably 10 ml or more, preferably 15 ml or more, preferably 20 ml or more, preferably 30 ml or more, preferably 50 ml or more, preferably 70 ml or more,
   (ii) 100 ml or less, preferably 70 ml or less, preferably 55 ml or less, preferably 25 ml or less, preferably 15 ml or less, preferably 10 ml or less, preferably 7 ml or less, preferably 6 ml or less, preferably 5 ml or less, preferably 4 ml or less, preferably 3 ml or less, preferably 1 ml or less, preferably 0.7 ml or less, preferably 0.5 ml or less, preferably 0.3 ml or less, preferably 0.2 ml or less,
      and/or
   (iii) between 0.1 ml and 100 ml, preferably between 0.1 ml and 1 ml or between 1 ml and 100 ml, such as between 1 ml and 50 ml, especially between 1 ml and 3 ml, between 1 ml and 10 ml, between 10 ml and 20 ml, between 20 ml and 30 ml, between 30 ml and 40 ml and/or between 40 ml and 50 ml.
Example 44. System according to any one of the preceding examples, wherein
   the position of the stopper moves 4.0 mm or less, preferably 3.0 mm or less, preferably 2.5 mm or less, preferably 2.0 mm or less, preferably 1.5 mm or less, preferably 1.0 mm or less, preferably 0.8 mm or less, preferably 0.5 mm or less, preferably 0.3 mm or less, preferably 0.1 mm or less, along the rotation axis of the barrel, when the system is cooled from room temperature (20°C) to -20°C, preferably -50°C, preferably -80°C.
Example 45. System according to any one of the preceding examples, wherein the system has a temperature of -20°C or less and -200°C or more, preferably -40°C or less and -120°C or more, preferably -50°C or less and -90°C or more.
Example 46. System according to any one of the preceding examples, wherein the system is stored for 5 days or more, preferably 1 month or more, preferably 3 month or more, preferably 6 month or more, preferably, 1 year or more.
Example 47. System according to any one of the preceding examples, wherein the stopper comprises at least one inner recess.
Example 48. System according to any one of the preceding examples, wherein the barrel comprises a polymer, preferably cyclic olefin copolymers (COC) and/or cyclic olefin polymers (COP).
Example 49. System according to any one of the preceding examples, wherein the closure comprises a brombutyl rubber, preferably wherein the closure is a siliconized closure comprising a brombutyl rubber.
Example 50. System according to any one of the preceding examples, wherein the barrel comprises a polymer, preferably COC and/or COP; and wherein the closure comprises a brombutyl rubber, preferably wherein the closure is a siliconized closure comprising a brombutyl rubber.
Example 51. Method comprising:
   Administering to a subject an effective amount of at least one pharmaceutically active ingredient comprised by a pharmaceutical composition using a system according to any one of the examples 1 to 47.
Example 52. Liquid composition for use
   in a method for treatment of the human or animal body by surgery or therapy, or
   in a diagnostic method practiced on the human or animal body,
   wherein the composition comprises at least one pharmaceutically active ingredient, and wherein the method comprises the step
   administering to a subject an effective amount of said active ingredient using a system according to any one of the examples 1 to 50.
Example 53. System according to any one of the examples 1 to 50 for use
   in a method for treatment of the human or animal body by surgery or therapy, or
   in a diagnostic method practiced on the human or animal body,
   wherein the composition comprises at least one pharmaceutically active ingredient, and wherein the method comprises the step
   administering to a subject an effective amount of said active ingredient using the system.
Example 54. System according to any one of the examples 1 to 50, method according to example 51, use according to example 52 and/or use according to example 53, wherein
   the composition comprises mRNA.
Example 55. Device for producing a system according to any one of the examples 1 to 50, the device comprising a servo motor and a laser fixedly attached, especially fixedly attached in a mechanically manner, to the servo motor.
Example 56. Method for producing a system according to any one of the examples 1 to 50, the method comprising (i) using a device according to example 55 and/or (ii) (a) providing a barrel, wherein preferably one end of the barrel is an open end and/or one further end of the barrel is a closed end, (b) filling the barrel with a liquid pharmaceutical composition, (c) providing a stopper, and/or (d) positioning the stopper by translating the stopper by means of a servo motor, especially the server motor comprised by the device, to a specific position within the barrel along the axial extension of the barrel, wherein the specific position is determined by means of a positioning system comprising a laser which laser is fixedly attached, especially fixedly attached in a mechanically manner, to the servo motor.
Example 57. Use of a system according to any one of the examples 1 to 50 for the long time storage at low temperature.
Example 58. Use according to example 57, wherein long time storage is 5 days or more, preferably 1 month or more, preferably 3 month or more, preferably 6 month or more, preferably, 1 year or more.
Example 59. Use according to any one of examples 57 to 58, wherein low temperature is -20 °C or less, preferably -50 °C or less, preferably -80 °C or less.
Example 60. Bundle comprising 5 or more, preferably 10 or more, preferably 30 or more, preferably 50 or more, preferably 100 or more, systems according to any one of the examples 1 to 47, wherein for each two of the systems, the volumes of the systems' headspaces, such as the systems' headspaces A and/or the systems' headspaces B, differ by not more than 0.5 ml, preferably by not more than 0.4 ml, preferably by not more than 0.3 ml, preferably by not more than 0.2 ml, preferably by not more than 0.1 ml.

### Examples

In the following table, two embodiments of an inventive system are compared against a conventional system known from the state of the art.

For each system there is provided the information concerning the volume occupied by the composition within the barrel (in ml, at room temperature), the height of the headspace within the barrel (in mm, at room temperature) and the (initial) volume occupied by the headspace within the barrel (in ml, at room temperature).

It has to be noted, that here the height of the headspace (in mm) is obtained from a system which is oriented such that the stopper is at the top and the closure is at the bottom. Then, the height of the headspace corresponds to the distance from the surface of the composition to the (flat) surface of the stopper faced towards the composition. The barrel of each of the systems has an inner diameter of 6.5 mm (SCHOTT TopPac^{®} 1 ml Ig).

It is particularly noted that the definition of the headspace used in this example is the definition of the headspace B and the height referred to in this example is the specific height, as, respectively, described elsewhere.

In addition, each system has been verified with an adapted CCI test. For the adapted CCI test, the system under test is cooled and thawed and the maximal movement of the stopper during this cycle is evaluated. To be more precise, for the adapted CCI test, the system is cooled from room temperature (20°C) to -50 °C during 12 hours with a constant cooling rate. Then, the system is kept at the temperature of -50 °C for 24 hours. Finally, the system is thawed from -50 °C to room temperature during 12 hours with a constant heating rate. The adapted CCI test is passed by the system, if the maximal movement of the stopper within the barrel is limited during cooling and thawing the system. Otherwise, it is failed by the system.

The maximum allowed movement is 3 mm for passing the adapted CCI test, since this is the maximal axial distance between two sealing lips of the systems' stopper.

In the table, for each system is also provided the maximal distance the stopper has moved during the adapted CCI test.

| | **Inventive System 1** | **Inventive System 2** | **Conventional System** |
|---|---|---|---|
| Volume composition | 1 ml | 1 ml | 1 ml |
| Height of Headspace | 2 mm | 6 mm | 0 mm |
| Volume head space | 0.066 ml | 0.199 ml | 0 ml |
| Max. movement of stopper | 2.35 mm | 1.05 mm | 3.85 mm |
| Adapted CCI test | Pass | Pass | Fail |

As can be taken from the table, the adapted CCI test is passed by the inventive system 1 and also by the inventive system 2. Here the maximal movement of the stopper during cooling and thawing is, respectively, 2.35 mm and 1.05 mm, which is less than the maximal allowed movement of 3 mm. However, the conventional system which has no headspace failed the adapted CCI test because of a maximal movement of the stopper of 3.85 mm.

In a further experiment, the influence of an additive was tested. Therefore, a syringe barrel (SCHOTT TopPac^{®} 1 ml Ig Syringe barrel closed with a Tipcap) was filled with an aqueous solution (0.75 ml) comprising an additive (see table) and then closed with a stopper (1 ml Ig West NovaPure ^{®}) so that the specific height was 2 mm. The position of the stopper was marked and afterwards, the system was cooled to -20°C (12 hours) in a freezer. Finally, the stopper movement was quantified directly after freezing.

| additive | Concentration (g/l) | Stopper movement (mm) |
|---|---|---|
| - | - | 1.54 |
| NaCl | 7.43 | 150 |
| NaCl | 14.85 | 1.24 |
| NaCl | 87 | 0.59 |
| sucrose | 87 | 130 |

Similar results were obtained with Dätwyler FM257, D6twyler FM457 and West FluroTec^{®} as stopper.

In a further experiment, the influence of the filling volume was tested. Therefore, a syringe barrel (SCHOTT TopPac^{®} 1 ml Ig Syringe barrel closed with a Tipcap) was filled with an aqueous solution (volume indicated in table below) comprising sucrose (87 g/l) and then closed with a stopper (1 ml Ig West NovaPure ^{®}) so that the specific height was 2 mm. The position of the stopper was marked and afterwards, the system was cooled to -20°C (12 hours) in a freezer. Finally, the stopper movement was quantified directly after freezing.

| Filing volume (ml) | Stopper movement (mm) |
|---|---|
| 0.25 | 000 |
| 0.50 | 1.10 |
| 0.75 | 130 |

In a further experiment, the influence of the specific height was tested. Therefore, a syringe barrel (syriQ^{®} sterile 1 ml long LL RF SRC W7025) was filled with an aqueous solution (water for injection, 0.5 ml) and then closed with a stopper (1 ml Ig West NovaPure ^{®}) so that the specific height was as indicated in the following table. The position of the stopper was marked and afterwards, the system was cooled to -80°C (12 hours) in a freezer. Finally, the stopper movement was quantified directly after freezing.

| **Specific height (mm)** | **Plunger movement (mm)** |
|---|---|
| 0 | 1.1 |
| 1 | 0.8 |
| 2 | 0.3^{t} |
| 4 | 0.4^{t} |
| 6 | 0.2^{t} |
| 10 | 0.4 |
| 15 | -2.0* |
| 20 | -3.1* |

| | |
|---|---|
| * "-" indicates that the stopper moves towards the closure, wherein in all other cases the stopper moves away from the closure ^{t} similar results were obtained using the stopper "1ml Dätwyler FM257" instead of the stopper "1 ml Ig West NovaPure ^{®}" (2 mm headspace: 0.4 mm plunger movement; 4 mm headspace: 0.2 mm plunger movement; 6 mm headspace: 0.0 mm plunger movement) | |

As can be seen, the best values were obtained for a specific height of 1 mm to 10 mm, particularly for 2 mm to 10 mm. A plunger movement of 0.7 mm or less was obtained in a similar experiment, in which the specific height was adjusted to 2 to 6 mm (0.5 or less for a specific height of 2 to 4 mm) and in which as syringe barrel syriQ^{®} sterile 1ml short LL RF SRC W7025 was used and as the stoppers 1-3 ml Dätwyler FM257 and 1-3 ml West NovaPure^{®} were used. Further, experiments were conducted using Dätwyler FM457 and West FluroTec^{®} and, in addition, using a comparable silicon free barrel and GORE^{™} ImproJect^{™} as stopper. These experiments show also similar results.

In a further experiment, the tightness of a closure during the freezing and storage of a syringe was tested. For this purpose, empty syringes were each closed with a stopper (here Datwyler, V9283 FM257/2 ISAF 2 067, Plunger 1 ml Ig FM257 ISAF 2) and a closure (here Datwyler V9317 FM257/2 ISAF1 002) and each stopper was further sealed with glue (here Loctite HY-SOL 3421 Liquid Epoxy Adhesive) to exclude a leakage at the stopper side. Afterwards, the CO₂ amount was measured by a FM-TDLAS (here lighthouse FMS-CO2; Serial Number 742). The samples were stored vertically in the -80°C freezer containing dry ice for 24 hours, and subsequently, the samples were let to thaw for at least 20 minutes prior to the measurement. The CO₂ amount was measured again by a FM-TDLAS (here lighthouse FMS-CO2; Serial Number 742). Two syringes were tested (here SCHOTT TopPac^{®} 1 ml Ig and syriQ^{®} sterile 1ml long LL RF SRC). In the glass syringe syriQ^{®} sterile 1ml long LL RF SRC, the CO₂ level was higher after freezing compared to the value obtained before freezing, whereas in the polymer syringe TopPac^{®} 1 ml Ig it was identical before and after freezing, indicating that no gas exchange occurred when a polymer syringe (here TopPac^{®} 1 ml Ig) and a closure (here Datwyler V9317 FM257/2 ISAF1 002) was used. The same trend was observed when using "Wild und Kupfer AG: TopPac^{®} Rigid Cap FM257, (ISAF 1)" as closure.

### Description of the Figures

The subject-matter of the present application is explained in more detail with reference to the subsequent figures without restricting said subject-matter to the shown embodiments, wherein
- Fig. 1: shows a system according to the first aspect of the invention;
- Fig. 2: shows parts of another system according to the first aspect of the invention;
- Fig. 3: shows another system according to the first aspect of the invention; and
- Fig. 4: shows a schematic illustration of a setup for carrying out a method according to the invention.

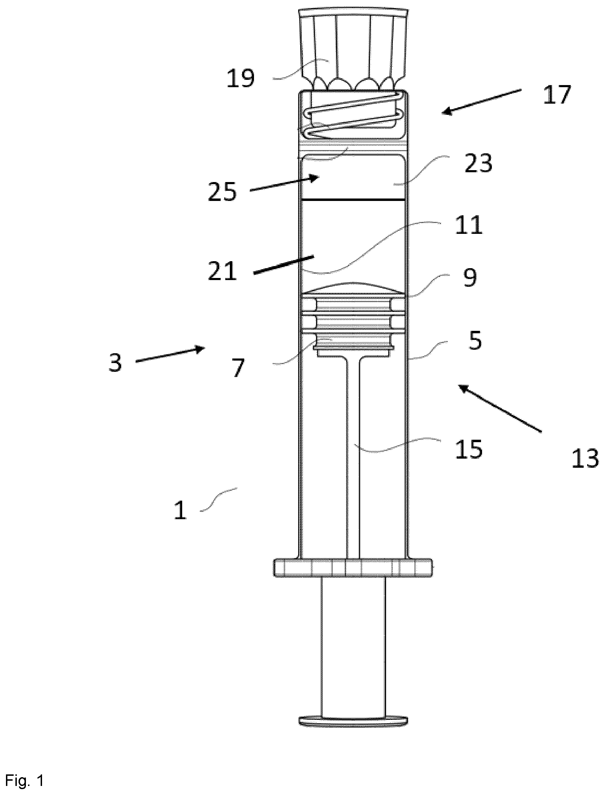

Figure 1 shows a system 1 for storing a pharmaceutical composition at low temperatures according to the first aspect of the invention. The system 1 comprises a pharmaceutical container 3. The container 3 has a barrel 5 and a stopper 7. The stopper 7 is slidably arranged within the barrel 5. The stopper 7 has a circumferential surface 9 (which is a surface of one of the sealing lips of the stopper 7) partially contacting the inner surface 11 of the barrel 5. The stopper 7 fluidally closes a first end 13 of the barrel 5. The stopper 7 is connected to a plunger rod 15. At its second end 17 the container 3 has threads for mounting an injection device (not shown) or a closure 19. The closure 19 fluidally closes the second end 17 of the barrel 5 and is further comprised by the container 3.

Within the barrel 5, between the stopper 7 and the closure 19, a liquid pharmaceutical composition 21 and a gas 23 are arranged. As shown in Fig. 1, the composition 21 and the gas 23 are arranged along the axial direction of the container 1. The volume enclosed by the barrel 5 between the stopper 7 and the closure 19 which is occupied by the gas 23 is defined as the headspace A 25 of the system 1.

At room temperature (293.15 K) the volume occupied by the headspace A 25 is 1 % or more of the volume occupied by the composition 21.

The system 1 is designed in such a way that in case of an expansion of the pharmaceutical composition 21, the pharmaceutical composition 21 can expand by reducing the volume of the headspace A 25. This is possible because the gas 23 can be displaced and/or compressed.

Hence, if the system 1 is cooled from room temperature (situation shown in Fig. 1) to below the freezing point of the composition 21, the stopper 7 does not move, even if the composition 21 takes more space due to expansion during cooling (at least at some instances of time during cooling). Instead, the composition 21 expands during cooling into the volume previously occupied by the gas 23 by compressing the gas 23. In other words: Its easier for the composition 21 to compress the gas 23 than moving the stopper 7 against the break loose force.

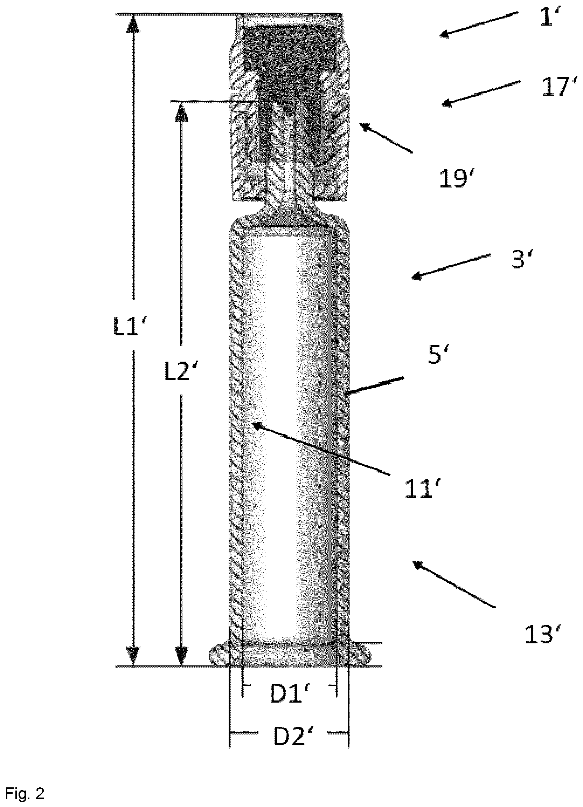

Figure 2 shows parts of another system 1' according to the first aspect of the invention in a perspective cut view. Structural features of the system 1' which are the same or similar to the features of system 1 described above with respect to Figure 1 are labeled with the same reference signs, however, single dashed. The system 1' is empty, i.e. no composition and no gas, and with removed stopper.

In Figure 2 the total length of the container 3' is indicated as L1'. And the total length of the barrel 5' is indicated as L2'. Furthermore, also the inside diameter D1' and the outside diameter D2' of the barrel 5' are indicated.

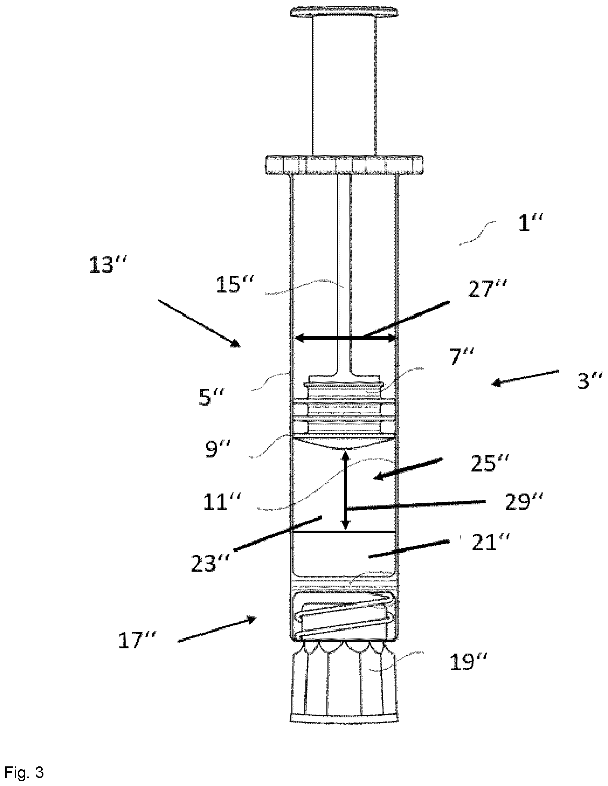

Figure 3 shows a system 1" which is similar to system 1 described above with respect to Figure 1. The system 1" in Fig. 3 has an orientation which is rotated by 180 degrees compared to that of system 1 in Fig. 1. Thus, for system 1" in Fig. 3, the first end 13" (i.e. which is closed by the stopper 7") is at the top and the second end 17" (i.e. which is closed by the closure 19") is at the bottom. Structural features of the system 1" which are the same or similar to the features of system 1 described above with respect to Figure 1 are labeled with the same reference signs, however, double dashed.

In Figure 3, the headspace B 25" can be identified. Apparently, the headspace B 25" has a cylindrical volume domain portion which volume domain portion has a specific diameter indicated by 27". The specific diameter 27" is equal to the inside diameter of the barrel 5". The volume domain portion has further a specific height indicated by 29".

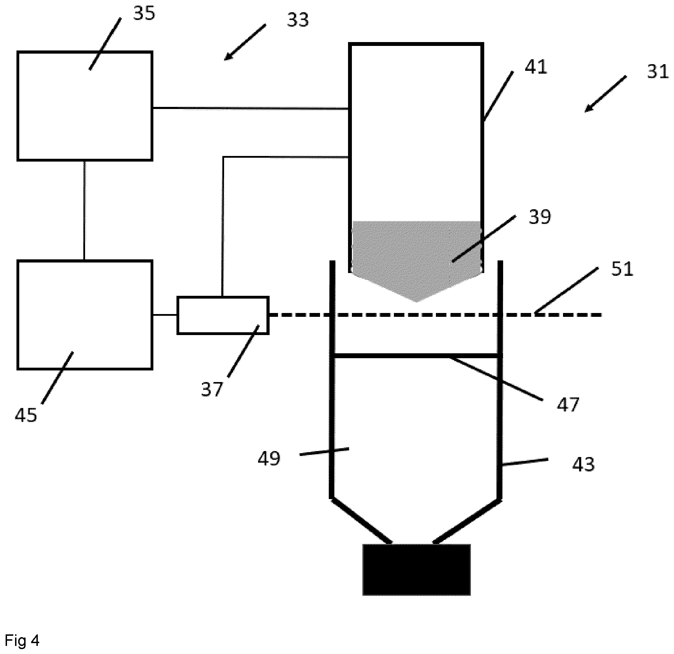

Figure 4 shows a schematic illustration of a setup 31 for carrying out a method according to aspects of the invention. The setup 31 comprises a device 33 for producing a system according to aspects of the invention, such as the system 1 described above with respect to Fig. 1.

The device 33 comprises a servo motor 35 and a laser 37 fixedly attached in a mechanically manner to the servo motor 35. In that the servo motor 35 is translated, also a stopper 39 can be translated by means of some kind of translating means 41 (which are part of the servo motor 35) along a vertical axis of a barrel 43. More precisely, the stopper 39 is attached to the translating means 41. And by translating the translating means 41, the stopper 39 can be positioned within the barrel 43.

When the stopper 39 is translated, also the laser 37 is translated in a synchronous manner, since it is fixedly attached to the servo motor 35. This allows for a positioning system 45, which may be coupled to and/or control the servo motor 35 and the laser 37, to detect the surface 47 of a liquid composition 49 hold within the barrel 43, once the surface 47 lines up with the laser beam 51 emitted by laser 37. Thus, the stopper 39 can be precisely positioned relative to the detected surface 47 of the liquid composition 49.

The method may comprise:
Providing the barrel 43, with one end of the barrel 43 is an open end and one further end of the barrel 43 is a closed end. In particular, the second end of the barrel 43 can be closed with the closure. Then, filling the barrel 43 with the liquid pharmaceutical composition 49 is conducted. Also comprised is providing the stopper 39 and positioning the stopper 39 by translating the stopper 39 by means of the servo motor 35 (especially the translating means 41) to a specific position within the barrel 43 along the axial extension of the barrel 43. Here, the specific position is determined by means of the positioning system 45 comprising the laser 37. The positioning can further comprise retracting the translating means and thereby releasing the stopper 39, which expands at the predetermined position within the barrel 43.

The features disclosed in the description, the figures as well as the claims could be essential alone or in every combination for the realization of the invention in its different embodiments.

### References

- 1, 1', 1": System
- 3, 3', 3": Pharmaceutical container
- 5, 5', 5": Barrel
- 7, 7": Stopper
- 9,9": Surface
- 11, 11', 11": Surface
- 13, 13', 13": First end
- 15, 15": Plunger rod
- 17, 17', 17": Second end
- 19, 19', 19": Closure
- 21, 21": Pharmaceutical composition
- 23, 23": Gas
- 25, 25": Headspace
- 27": Diameter
- 29": Specific Height
- 31: Setup
- 33: Device
- 35: Servo motor
- 37: Laser
- 39: Stopper
- 41: Translating means
- 43: Barrel
- 45: Positioning system
- 47: Surface
- 49: Liquid composition
- L1', L2': Length
- D1', D2': Diameter

## Claims

1. System for storing a pharmaceutical composition at low temperatures, the system comprising
- a pharmaceutical container for storing a pharmaceutical composition at low temperatures, the container having a barrel, a stopper, which fluidally closes a first end of the barrel, and a closure, which fluidally closes a second end of the barrel;
- a liquid pharmaceutical composition and a gas, both, arranged within the barrel of the container between the stopper and the closure;
wherein the liquid pharmaceutical composition comprises an additive.

2. System according to claim 1, wherein
the additive is selected from a group consisting of NaCl, KCI, sucrose, sodium acetate, acetic acid, ((4-hydroxybutyl)azanediyl)bis(hexan-6,1-diyl)bis(2-hexyldecanoat), 2[(polyethylene glycol)-2000]-N,N-ditetradecylacetamide, 1,2-Distearoyl-sn-glycero-3-phosphocholine, SM(sphyngomyelin)-102, polyethylene glycol [PEG] 2000 dimyristoyl glycerol [DMG], 1,2-distearoyl-sn-glycero-3-phosphocholine [DSPC], cholesterol, polyethylene glycol (PEG), H₃PO₄, XH₂PO₄, X₂HPO₄ and X₃PO₄, wherein X is Na and/or K.

3. System according to any one of the preceding claims, wherein (i) the additive is NaCl, KCI, sucrose, sodium acetate, acetic acid, polyethylene glycol (PEG), cholesterol and/or H₃PO₄ and/or (ii) the additive is selected from a group consisting of salt, sugar, lipid and acid.

4. System according to any one of the preceding claims, wherein (i) the additive is a lipid, preferably ((4-hydroxybutyl)azanediyl)bis(hexan-6,1-diyl)bis(2-hexyldecanoat), 2[(polyethylene glycol)-2000]-N,N-ditetradecylacetamide, 1,2-Distearoyl-sn-glycero-3-phosphocholine, SM(sphyngomyelin)-102, polyethylene glycol [PEG] 2000 dimyristoyl glycerol [DMG], 1,2-distearoyl-sn-glycero-3-phosphocholine [DSPC] and/or cholesterol, and/or (ii) the additive is XH₂PO₄, wherein X is Na and/or K, is X₂HPO₄, wherein X is Na and/or K, and/or is X₃PO₄, wherein X is Na and/or K.

5. System according to any one of the preceding claims, wherein the concentration of the additive is:
(i) 0.01 mol/l or more, preferably 0.05 mol/l or more, preferably 0.1 mol/l or more, preferably 0.2 mol/l or more, preferably 0.3 mol/l or more, preferably 0.4 mol/l or more, preferably 0.5 mol/l or more, preferably 0.7 mol/l or more,
(ii) 1 mol/l or less,
and/or
(iii) between 0.1 mol/l and 1 mol/l.

6. System according to any one of the preceding claims, wherein the concentration of all compounds in the pharmaceutical composition is
(i) 0.01 mol/l or more, preferably 0.05 mol/l or more, preferably 0.1 mol/l or more, preferably 0.2 mol/l or more, preferably 0.3 mol/l or more, preferably 0.4 mol/l or more, preferably 0.5 mol/l or more, preferably 0.7 mol/l or more,
(ii) 1 mol/l or less,
and/or
(iii) between 0.1 mol/l and 1 mol/l.

7. System according to any one of the preceding claims, wherein
(i) the storage temperature is
(a) -100 degrees C or above, preferably -80 degrees C or above, preferably -60 degrees C or above, preferably -40 degrees C or above, preferably -20 degrees C or above,
(b) 0 degrees C or below, -1 degrees C or below, preferably -5 degrees C or below, preferably -15 degrees C or below,
and/or
(c) between -100 and 0 degrees C;
and/or
(ii) the storage temperature is below the freezing temperature of the composition.

8. System according to any one of the preceding claims, wherein
(i) the pharmaceutical composition comprises H₂O, especially more than 80 % (w/w), preferably more than 90 % (w/w), preferably more than 95 % (w/w), preferably more than 97 % (w/w), preferably more than 98 % (w/w), of the composition is H₂O;
and/or
(ii) the gas comprises or is air, CO₂, N₂, Ar and/or O₂.

9. System according to any one of the preceding claims, wherein
at room temperature the composition has a volume of
(i) 0.1 ml or more, preferably 0.2 ml or more, preferably 0.3 ml or more, preferably 0.5 ml or more, preferably 0.7 ml or more, preferably 1 ml or more, preferably 1.5 ml or more, preferably 2 ml or more, preferably 3 ml or more, preferably 5 ml or more, preferably 10 ml or more, preferably 15 ml or more, preferably 20 ml or more, preferably 30 ml or more, preferably 50 ml or more, preferably 70 ml or more,
(ii) 100 ml or less,
and/or
(iii) between 0.1 ml and 100 ml.

10. System according to any one of the preceding claims, wherein
the position of the stopper moves 4.0 mm or less, preferably 3.0 mm or less, preferably 2.5 mm or less, preferably 2.0 mm or less, preferably 1.5 mm or less, preferably 1.0 mm or less, preferably 0.8 mm or less, preferably 0.5 mm or less, preferably 0.3 mm or less, preferably 0.1 mm or less, along the rotation axis of the barrel, when the system is cooled from room temperature (20°C) to -20°C, preferably -50°C, preferably -80°C.

11. System according to any one of the claims 1 to 10, wherein the composition comprises mRNA.

12. Use of a system according to any one of the claims 1 to 11 for the long time storage at low temperature, wherein preferably (i) long time storage is 5 days or more, preferably 1 month or more, preferably 3 month or more, preferably 6 month or more, preferably, 1 year or more and/or (ii) low temperature is -20 °C or less, preferably -50 °C or less, preferably -80 °C or less.
